# EUROPEAN PATENT SPECIFICATION

(11) **EP 4 247 463 B1**
(45) Date of publication and mention of the grant of the patent: **30.09.2026**
(21) Application number: 21893150.9
(22) Date of filing: 19.11.2021
(51) Int. Cl.: A61M 16/06, A61M 16/20

(54) **VENT AND AAV ARRANGEMENT FOR PATIENT INTERFACE**
ENTLÜFTUNG UND AAV-ANORDNUNG FÜR EINE PATIENTENSCHNITTSTELLE
DISPOSITIF DE VENTILATION ET DE SOUPAPE D'ADMISSION D'AIR POUR INTERFACE PATIENT

(30) Priority: 20.11.2020 US 202063116202 P
(43) Date of publication of application: 27.09.2023
(73) Proprietor: ResMed Pty Ltd, Bella Vista, New South Wales 2153 (AU)
(72) Inventor: SHANMUGA SUNDARA, Shiva Kumar, Bella Vista, New South Wales 2153 (AU); MARKAPURAM CHENGALVARAYAN, Kishore, Bella Vista, New South Wales 2153 (AU)
(74) Representative: Vossius & Partner Patentanwälte Rechtsanwälte mbB
(86) International application number: PCT/AU2021/051384
(87) International publication number: WO 2022/104432

(56) References cited:
- WO-A1-2009/108995
- WO-A1-2019/119058
- WO-A1-2020/240462
- JP-B2- 6 622 873
- US-A1- 2016 129 214
- US-A1- 2019 091 432
- US-A1- 2020 046 933
- US-A1- 2020 353 198

## Description

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

### 1 CROSS-REFERENCE TO RELATED APPLICATIONS

This application claims the benefit of U.S. Provisional Application No. 63/116,202, filed November 20, 2020.

### 2 BACKGROUND OF THE TECHNOLOGY

### 2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 2.2 DESCRIPTION OF THE RELATED ART

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 2.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFT^{™} nasal pillows mask, SWIFT^{™} II nasal pillows mask, SWIFT^{™} LT nasal pillows mask, SWIFT^{™} FX nasal pillows mask and MIRAGE LIBERTY^{™} full-face mask. The following patent applications, assigned to ResMed Limited, describe examples of nasal pillows masks: International Patent Application WO2004/073,778 (describing amongst other things aspects of the ResMed Limited SWIFT^{™} nasal pillows), US Patent Application 2009/0044808 (describing amongst other things aspects of the ResMed Limited SWIFT^{™} LT nasal pillows); International Patent Applications WO 2005/063,328 and WO 2006/130,903 (describing amongst other things aspects of the ResMed Limited MIRAGE LIBERTY^{™} full-face mask); International Patent Application WO 2009/052,560 (describing amongst other things aspects of the ResMed Limited SWIFT^{™} FX nasal pillows).

### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 cmH₂O).

| RPT Device name | A-weighted sound pressure level dB(A) | Year (approx.) |
|---|---|---|
| C-Series Tango^{™} | 31.9 | 2007 |
| C-Series Tango^{™} with Humidifier | 33.1 | 2007 |
| S8 Escape^{™} II | 30.5 | 2005 |
| S8 Escape^{™} II with H4i^{™} Humidifier | 31.1 | 2005 |
| S9 AutoSet^{™} | 26.5 | 2010 |
| S9 AutoSet^{™} with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar^{™} Series of Adult and Paediatric Ventilators may provide support for invasive and non-invasive non-dependent ventilation for a range of patients for treating a number of conditions such as but not limited to NMD, OHS and COPD.

The ResMed Elisée^{™} 150 ventilator and ResMed VS III^{™} ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

A range of artificial humidification devices and systems are known, however they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore, medical humidifiers may have more stringent safety constraints than industrial humidifiers

While a number of medical humidifiers are known, they can suffer from one or more shortcomings. Some medical humidifiers may provide inadequate humidification, some are difficult or inconvenient to use by patients.

### 2.2.3.5 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 2.2.3.6 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

**Table of noise of prior masks (ISO 17510-2:2007, 10 cmH₂O pressure at 1m)**

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage^{™} (*) | nasal | 29.5 | 21.5 | 1998 |
| ResMed UltraMirage^{™} | nasal | 36 (3) | 28 (3) | 2000 |
| ResMed Mirage Activa^{™} | nasal | 32 (3) | 24 (3) | 2002 |
| ResMed Mirage Micro^{™} | nasal | 30 (3) | 22 (3) | 2008 |
| ResMed Mirage^{™} SoftGel | nasal | 29 (3) | 22 (3) | 2008 |
| ResMed Mirage^{™} FX | nasal | 26 (3) | 18 (3) | 2010 |
| ResMed Mirage Swift^{™} (*) | nasal pillows | 37 | 29 | 2004 |
| ResMed Mirage Swift^{™} II | nasal pillows | 28 (3) | 20 (3) | 2005 |
| ResMed Mirage Swift^{™} LT | nasal pillows | 25 (3) | 17 (3) | 2008 |
| ResMed AirFit P10 | nasal pillows | 21 (3) | 13 (3) | 2014 |

| | | | | |
|---|---|---|---|---|
| (* one specimen only, measured using test method specified in ISO 3744 in CPAP mode at 10 cmH₂O) | | | | |

Sound pressure values of a variety of objects are listed below

| Object | A-weighted sound pressure dB(A) | Notes |
|---|---|---|
| Vacuum cleaner: Nilfisk Walter Broadly Litter Hog: B+ Grade | 68 | ISO 3744 at 1m distance |
| Conversational speech | 60 | 1m distance |
| Average home | 50 | |
| Quiet library | 40 | |
| Quiet bedroom at night | 30 | |
| Background in TV studio | 20 | |

### 3 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

Another aspect of the present technology is directed to a patient interface that may comprise: a plenum chamber; a seal-forming structure; and a positioning and stabilising structure. The patient interface may further comprise a vent structure. The patient interface may further be configured to leave the patient's mouth uncovered, or if the seal-forming structure is configured to seal the patient's nose and mouth, the patient interface may be further configured to allow the patient to breath from ambient in the absence of a flow of pressurised air.

Another aspect of the present technology is directed to a patient interface comprising: a plenum chamber pressurisable to a therapeutic pressure of at least 4 cmH2O above ambient air pressure, said plenum chamber including a plenum chamber inlet port sized and structured to receive a flow of air at the therapeutic pressure for breathing by a patient; a seal-forming structure constructed and arranged to seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use; a positioning and stabilising structure configured to hold the seal-forming structure in a therapeutically effective position on the patient's head, the positioning and stabilising structure comprising a tie, the tie being constructed and arranged so that at least a portion overlies a region of the patient's head superior to an otobasion superior of the patient's head in use; and a vent structure configured to allow a continuous flow of gases exhaled by the patient from an interior of the plenum chamber to ambient, said vent structure being sized and shaped to maintain the therapeutic pressure in the plenum chamber in use; wherein the patient interface is configured to leave the patient's mouth uncovered, or if the seal-forming structure is configured to seal around the patient's nose and mouth, the patient interface is configured to allow the patient to breath from ambient in the absence of a flow of pressurised air.

An aspect of the present technology relates to a patient interface including a vent to discharge gas exhaled by the patient. The patient interface may comprise a diffusing member structured and arranged to diffuse the vent flow to produce less noise. The patient interface may include an anti-asphyxia valve (AAV) configured to allow the patient to breathe in ambient air and exhale if pressurized gas is not of sufficient magnitude or not delivered.

Another aspect of the present technology relates to a patient interface to deliver a flow of air at a positive pressure with respect to ambient air pressure to an entrance to the patient's airways including at least the entrance of a patient's nares while the patient is sleeping, to ameliorate sleep disordered breathing. The patient interface includes a seal-forming structure constructed and arranged to form a seal with a region of a patient's face surrounding the entrance to the patient's airways, the seal-forming structure forming at least a portion of a plenum chamber pressurizable to a therapeutic pressure, and a vent and AAV arrangement. The vent and AAV arrangement is configured to regulate flow therethrough to (1) provide a vent flow path when pressure in the plenum chamber is above a predetermined magnitude and (2) provide a breathable flow path when pressure in the plenum chamber is below the predetermined magnitude or not delivered. The vent and AAV arrangement includes a shell portion including a port extending therethrough, the port configured to allow gas to flow between the plenum chamber and ambient, an AAV member provided to the shell portion, the AAV member including a flap portion structured and arranged to regulate flow through the port, a diffusing member provided to the shell portion, and a diffusing member cover to retain the diffusing member to the shell portion. The flap portion includes a plurality of vent holes therethrough. The flap portion is movable to a deactivated position when pressure in the plenum chamber is above the predetermined magnitude to cover the port so that a vent flow of gas is allowed to pass along the vent flow path that extends through the plurality of vent holes of the flap portion and through the port. The flap portion is movable to an activated position when pressure in the plenum chamber is below the predetermined magnitude or not delivered to uncover the port so that a breathable flow of gas is allowed to pass along the breathable flow path that extends through the port. The diffusing member is configured and arranged such that the port is covered by the diffusing member so that at least a portion of the vent flow of gas passes into the diffusing member. The diffusing member cover includes an anterior wall completely covering an anterior side of the diffusing member. The diffusing member and diffusing member cover are supported in spaced relation from an outlet end of the port to form a lateral opening between the shell portion and the diffusing member and diffusing member cover, and at least a portion of the vent flow of gas passes into the diffusing member along the vent flow path and then is redirected laterally by the anterior wall to flow through the lateral opening to ambient.

Another aspect of the present technology relates to a patient interface to deliver a flow of air at a positive pressure with respect to ambient air pressure to an entrance to the patient's airways including at least the entrance of a patient's nares while the patient is sleeping, to ameliorate sleep disordered breathing. The patient interface includes a seal-forming structure constructed and arranged to form a seal with a region of a patient's face surrounding the entrance to the patient's airways, the seal-forming structure forming at least a portion of a plenum chamber pressurizable to a therapeutic pressure, and a vent and AAV arrangement. The vent and AAV arrangement is configured to regulate flow therethrough to (1) provide a vent flow path when pressure in the plenum chamber is above a predetermined magnitude and (2) provide a breathable flow path when pressure in the plenum chamber is below the predetermined magnitude or not delivered. The vent and AAV arrangement includes a shell portion including a first port and a second port spaced apart from the first port, each of the first port and the second port extending through the shell portion and configured to allow gas to flow between the plenum chamber and ambient, a first AAV member provided to the shell portion, a second AAV member provided to the shell portion, and a plurality of vent holes provided to the shell portion. The first AAV member includes a first flap portion structured and arranged to regulate flow through the first port and the second AAV member includes a second flap portion structured and arranged to regulate flow through the second port. Each of the first flap portion and the second flap portion is movable to a deactivated position when pressure in the plenum chamber is above the predetermined magnitude to cover respective ones of the first and second ports and prevent flow through respective ones of the first and second ports. Each of the first flap portion and the second flap portion is movable to an activated position when pressure in the plenum chamber is below the predetermined magnitude or not delivered to uncover respective ones of the first and second ports so that a breathable flow of gas is allowed to pass along the breathable flow path that extends through respective ones of the first and second ports. The plurality of vent holes are arranged between and spaced apart from the first and second ports, and a vent flow of gas is allowed to pass along the vent flow path that extends through the plurality of vent holes when pressure in the plenum chamber is above the predetermined magnitude.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a patient interface that may be washed in a home of a patient, e.g., in soapy water, without requiring specialised cleaning equipment.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 4 BRIEF DESCRIPTION OF THE DRAWINGS

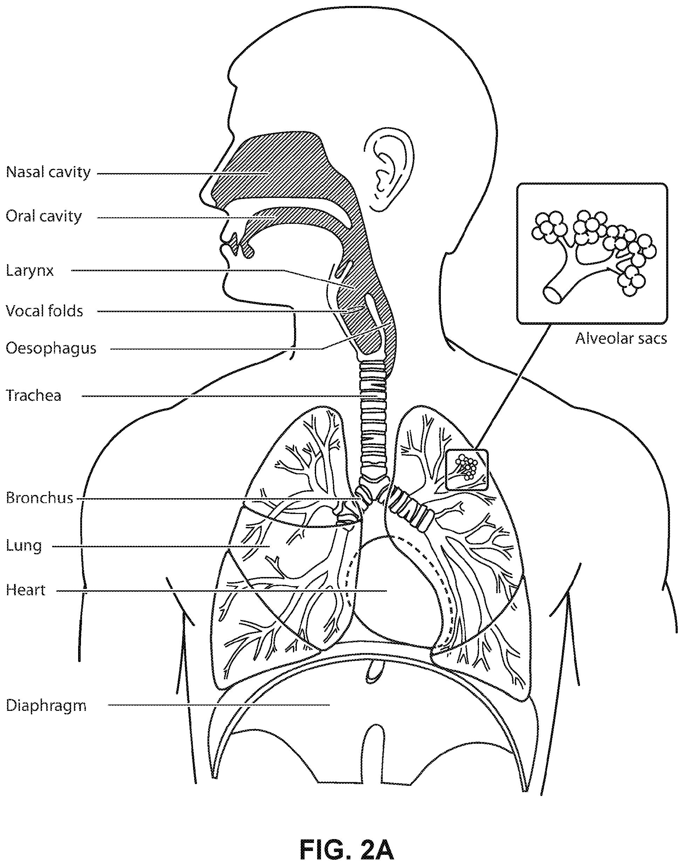

The present technology is illustrated by way of example, and not by way of limitation, in the figures of the accompanying drawings, in which like reference numerals refer to similar elements including:
4.1 RESPIRATORY THERAPY SYSTEMS
   Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.
   Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.
   Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.
4.2 RESPIRATORY SYSTEM AND FACIAL ANATOMY
   Fig. 2A shows an overview of a human respiratory system including the nasal and oral cavities, the larynx, vocal folds, oesophagus, trachea, bronchus, lung, alveolar sacs, heart and diaphragm.

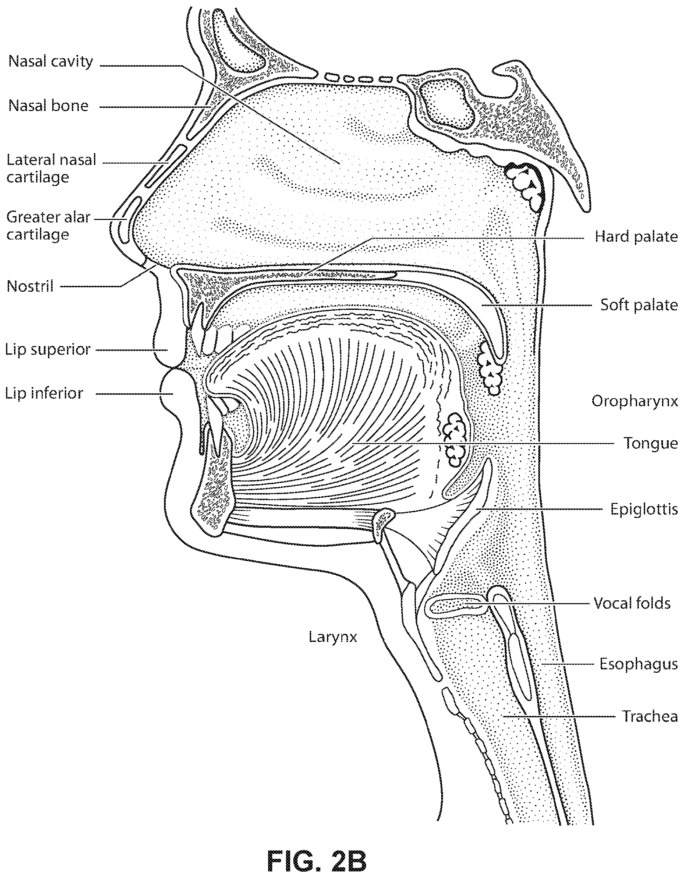

   Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

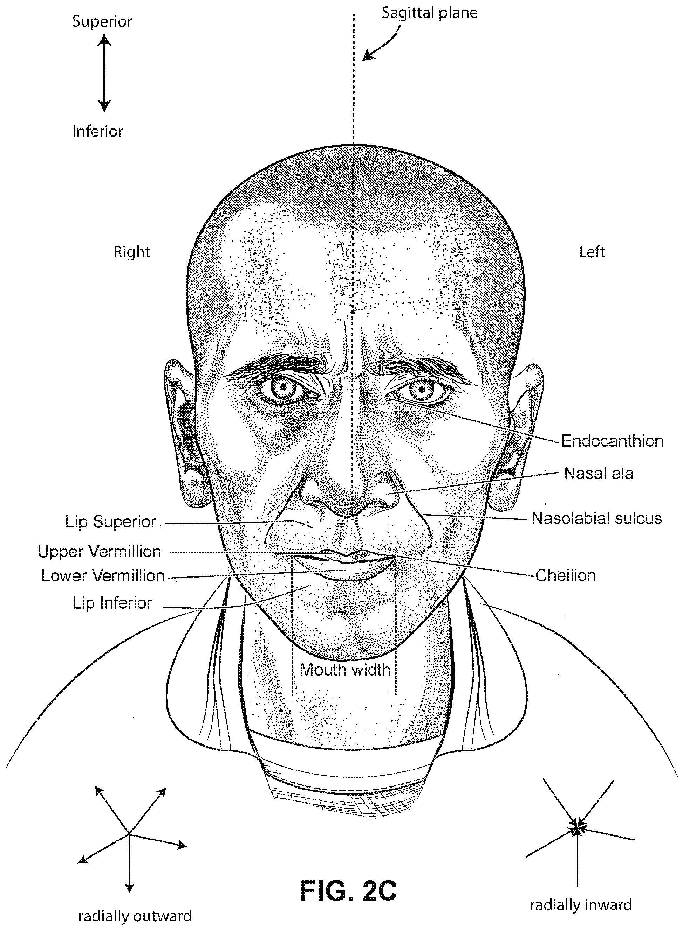

   Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

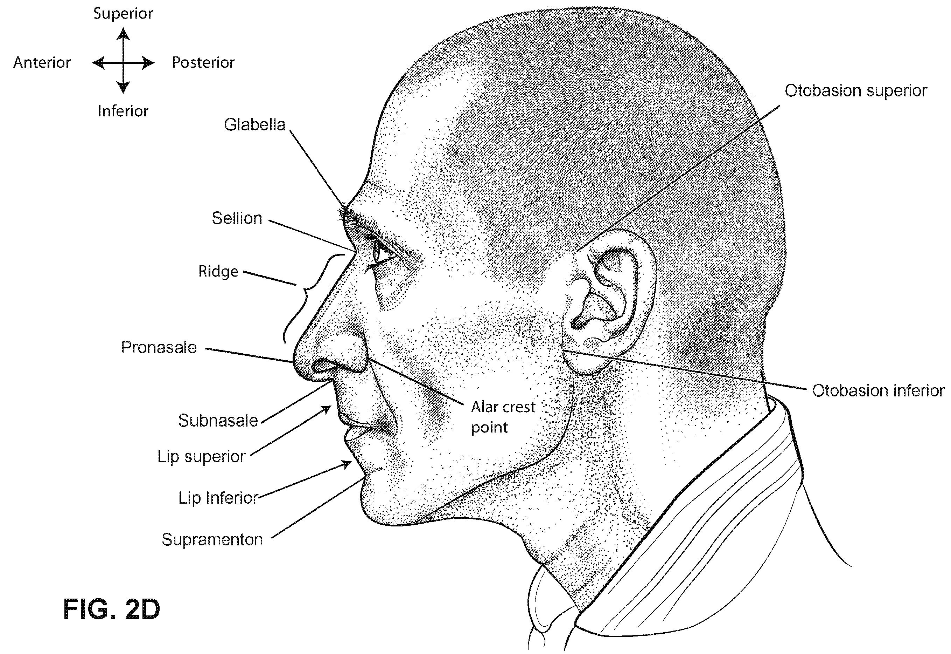

   Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

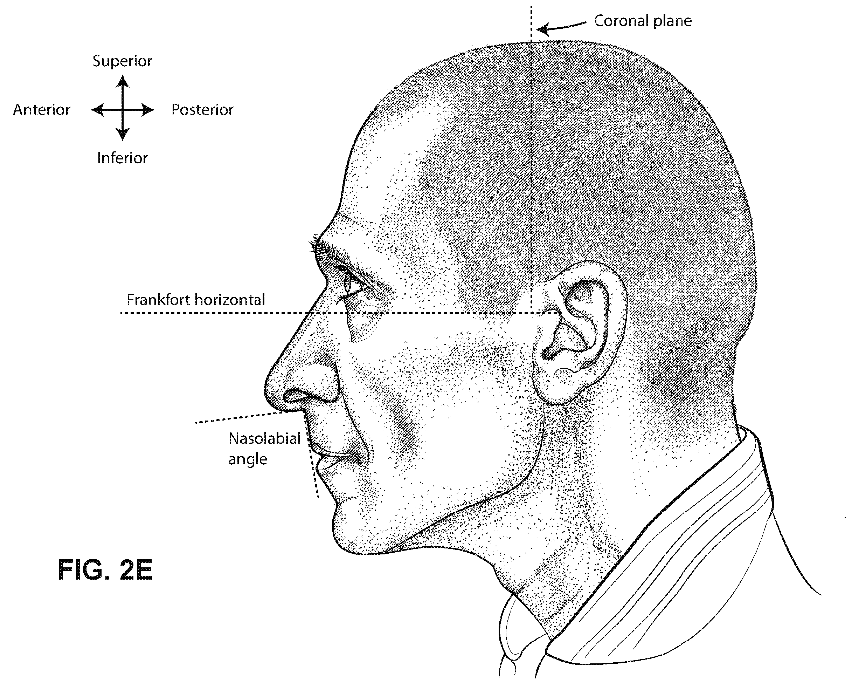

   Fig. 2E is a further side view of a head. The approximate locations of the Frankfort horizontal and nasolabial angle are indicated. The coronal plane is also indicated.

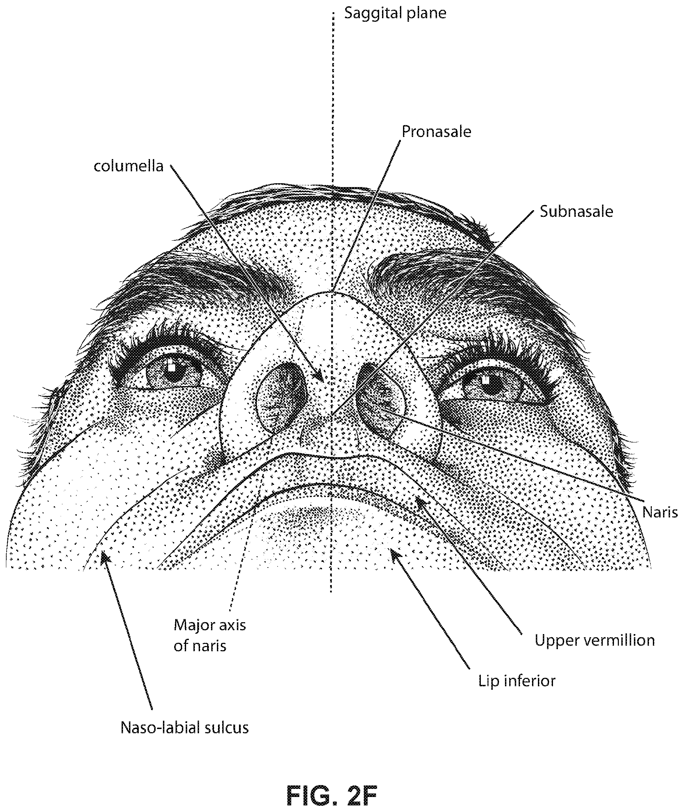

   Fig. 2F shows a base view of a nose with several features identified including naso-labial sulcus, lip inferior, upper Vermilion, naris, subnasale, columella, pronasale, the major axis of a naris and the midsagittal plane.

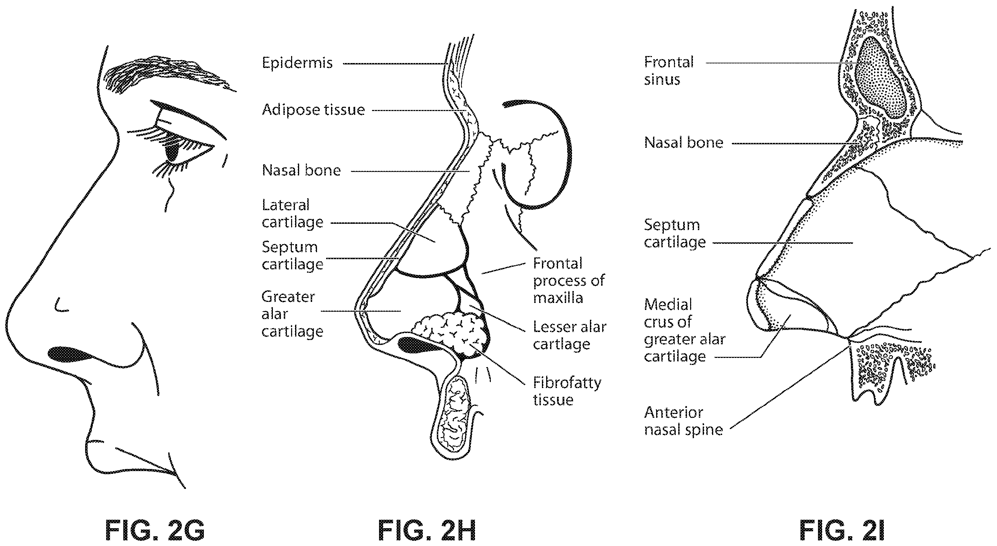

   Fig. 2G shows a side view of the superficial features of a nose.
   Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
   Fig. 2I shows a medial dissection of a nose, approximately several millimeters from the midsagittal plane, amongst other things showing the septum cartilage and medial crus of greater alar cartilage.

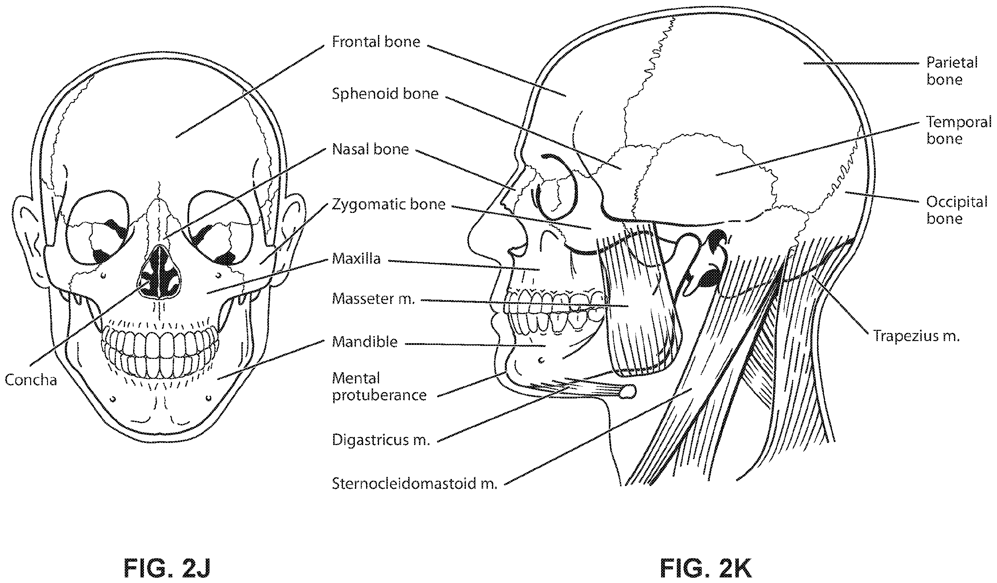

   Fig. 2J shows a front view of the bones of a skull including the frontal, nasal and zygomatic bones. Nasal concha are indicated, as are the maxilla, and mandible.
   Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

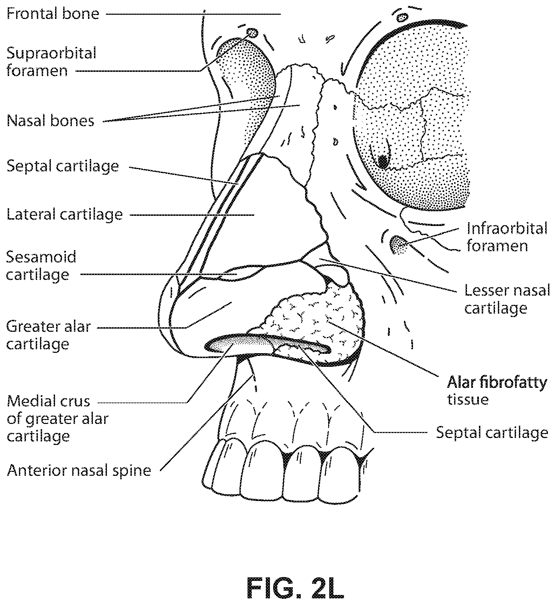

   Fig. 2L shows an anterolateral view of a nose.
4.3 PATIENT INTERFACE
   Fig. 3A shows a patient interface in the form of a nasal mask in accordance with one form of the present technology.
   Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
   Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
   Fig. 3D shows a schematic of a cross-section through a structure at a point. An outward normal at the point is indicated. The curvature at the point has a value of zero.
   Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
   Fig. 3F shows a schematic of a cross-section through a structure at a point. An outward normal at the point is indicated. The curvature at the point has a negative sign, and a relatively large magnitude when compared to the magnitude of the curvature shown in Fig. 3E.
   Fig. 3G shows a cushion for a mask that includes two pillows. An exterior surface of the cushion is indicated. An edge of the surface is indicated. Dome and saddle regions are indicated.
   Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.
   Fig. 3I shows the surface of a structure, with a one dimensional hole in the surface. The illustrated plane curve forms the boundary of a one dimensional hole.
   Fig. 3J shows a cross-section through the structure of Fig.3I. The illustrated surface bounds a two dimensional hole in the structure of Fig. 3I.
   Fig. 3K shows a perspective view of the structure of Fig. 3I, including the two dimensional hole and the one dimensional hole. Also shown is the surface that bounds a two dimensional hole in the structure of Fig. 3I.
   Fig. 3L shows a mask having an inflatable bladder as a cushion.
   Fig. 3M shows a cross-section through the mask of Fig. 3L, and shows the interior surface of the bladder. The interior surface bounds the two dimensional hole in the mask.
   Fig. 3N shows a further cross-section through the mask of Fig. 3L. The interior surface is also indicated.
   Fig. 3O illustrates a left-hand rule.
   Fig. 3P illustrates a right-hand rule.
   Fig. 3Q shows a left ear, including the left ear helix.
   Fig. 3R shows a right ear, including the right ear helix.
   Fig. 3S shows a right-hand helix.
   Fig. 3T shows a view of a mask, including the sign of the torsion of the space curve defined by the edge of the sealing membrane in different regions of the mask.
   Fig. 3U shows a view of a plenum chamber 3200 showing a sagittal plane and a mid-contact plane.
   Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

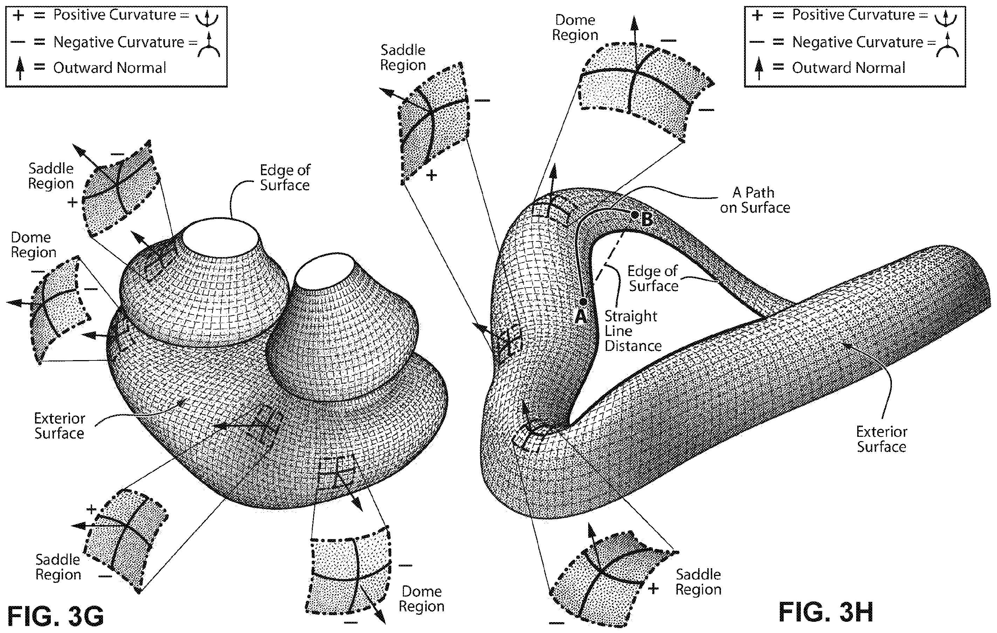

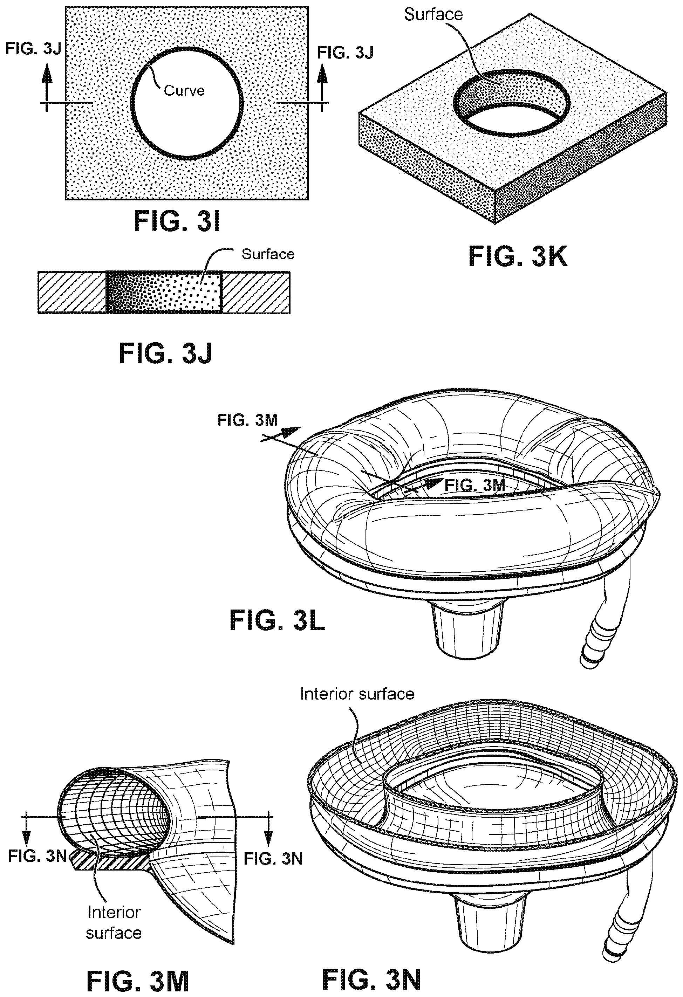

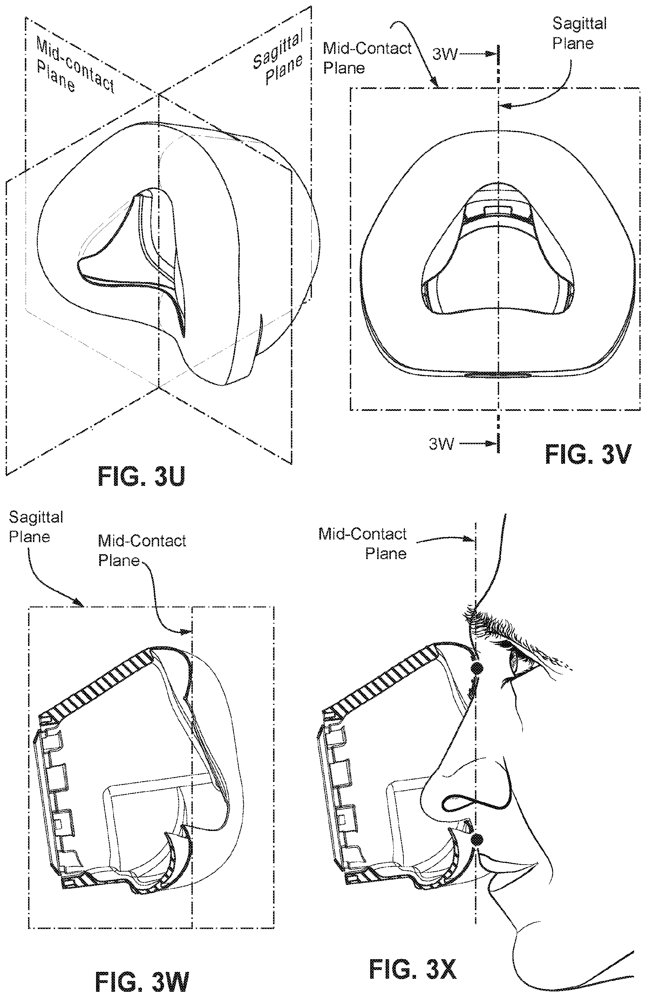

   Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.
   Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.
   Fig. 4 shows a patient interface in accordance with another form of the present technology.
4.4 VENT AND AAV ARRANGEMENT
   Fig. 5A is a perspective view of a patient interface including a vent and AAV arrangement according to an example of the present technology.
   Fig. 5B is a front view of the vent and AAV arrangement of Fig. 5A, with the diffusing member and the diffusing member cover removed according to an example of the present technology.
   Fig. 5C is a perspective view of the vent and AAV arrangement of Fig. 5A, with the diffusing member and the diffusing member cover removed according to an example of the present technology.
   Fig. 5D is a cross-sectional view of the vent and AAV arrangement of Fig. 5A according to an example of the present technology.
   Fig. 5E is another cross-sectional view of the vent and AAV arrangement of Fig. 5A according to an example of the present technology.
   Fig. 5F is another cross-sectional view of the vent and AAV arrangement of Fig. 5A according to an example of the present technology.
   Fig. 5G is another cross-sectional view of the vent and AAV arrangement of Fig. 5A according to an example of the present technology.
   Fig. 5H is an exploded view of a vent and AAV arrangement and shell of a patient interface according to an example of the present technology.
   Fig. 5I is a perspective view of an AAV member of the vent and AAV arrangement of Fig. 5A.
   Fig. 5J is a cross-sectional view showing the vent and AAV arrangement of Fig. 5A when pressure in the patient interface is below a predetermined magnitude or not delivered according to an example of the present technology.
   Fig. 5K is a cross-sectional view showing the vent and AAV arrangement of Fig. 5A when pressure in the patient interface is above a predetermined magnitude according to an example of the present technology.
   Fig. 6A is a front view of a patient interface including a vent and AAV arrangement according to another example of the present technology, with the diffusing member and the diffusing member cover removed.
   Fig. 6B is a top view of an AAV member of the vent and AAV arrangement of Fig. 6A.
   Fig. 7A is a perspective view of a patient interface including a vent and AAV arrangement according to another example of the present technology.
   Fig. 7B is a front view of the vent and AAV arrangement of Fig. 7A.
   Fig. 7C is a front view of the vent and AAV arrangement of Fig. 7A, with the diffusing member and the diffusing member cover removed according to an example of the present technology.
   Fig. 7D is a front perspective view of the vent and AAV arrangement of Fig. 7A.
   Fig. 7E is a perspective view of the vent and AAV arrangement of Fig. 7A.
   Fig. 7F is a top view of an AAV member of the vent and AAV arrangement of Fig. 7A.
   Fig. 7G is a top view of a diffusing member cover of the vent and AAV arrangement of Fig. 7A.
   Fig. 8A is a perspective view of a patient interface including a vent and AAV arrangement according to another example of the present technology.
   Fig. 8B is a cross-sectional view showing the vent and AAV arrangement of Fig. 8A.
   Fig. 8C is a cross-sectional view showing the vent and AAV arrangement of Fig. 8A when pressure in the patient interface is below a predetermined magnitude or not delivered according to an example of the present technology.
   Fig. 9A is a perspective view of a patient interface including a vent and AAV arrangement according to another example of the present technology.
   Fig. 9B is a front view of the vent and AAV arrangement of Fig. 9A.
   Fig. 9C is a rear perspective view of the vent and AAV arrangement of Fig. 9A.

### DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 5.2 RESPIRATORY THERAPY SYSTEMS

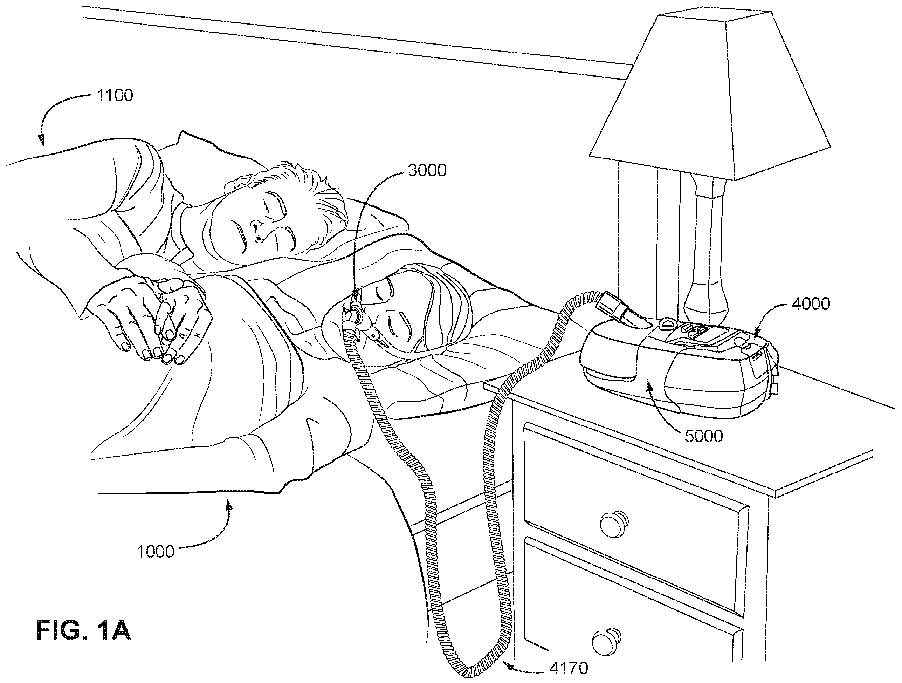

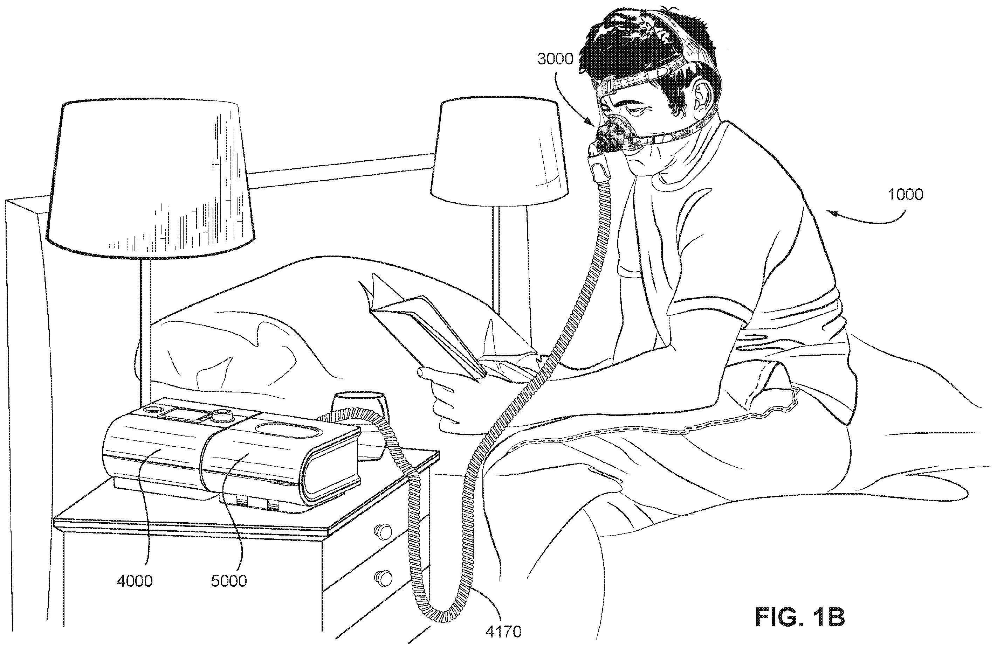

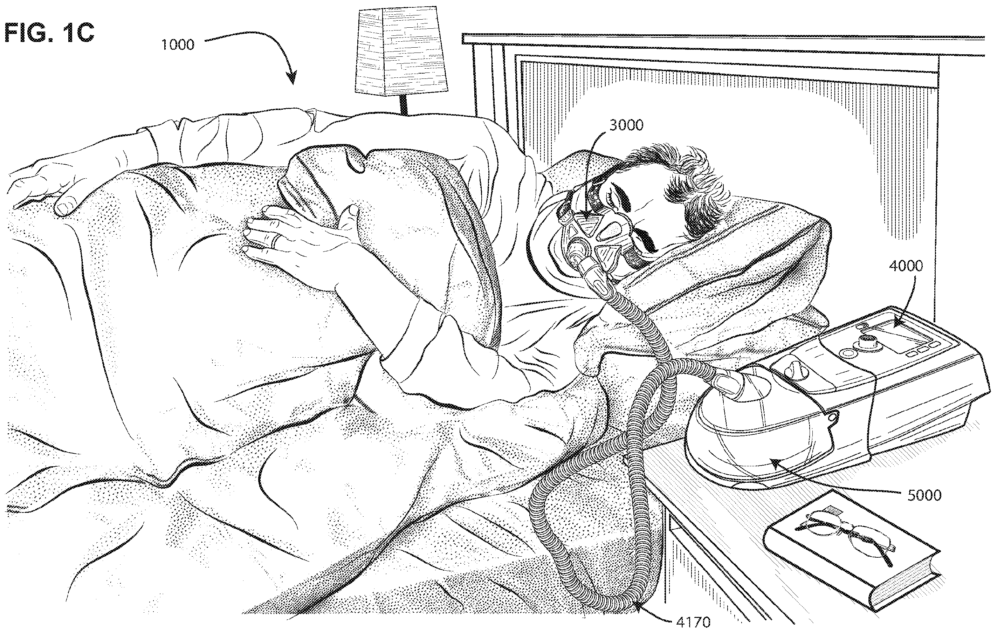

In one form, the present technology comprises a respiratory therapy system for treating a respiratory disorder. The respiratory therapy system may comprise an RPT device 4000 for supplying a flow of air to the patient 1000 via an air circuit 4170 and a patient interface, e.g., see Figs. 1A to 1C.

### 5.3 PATIENT INTERFACE

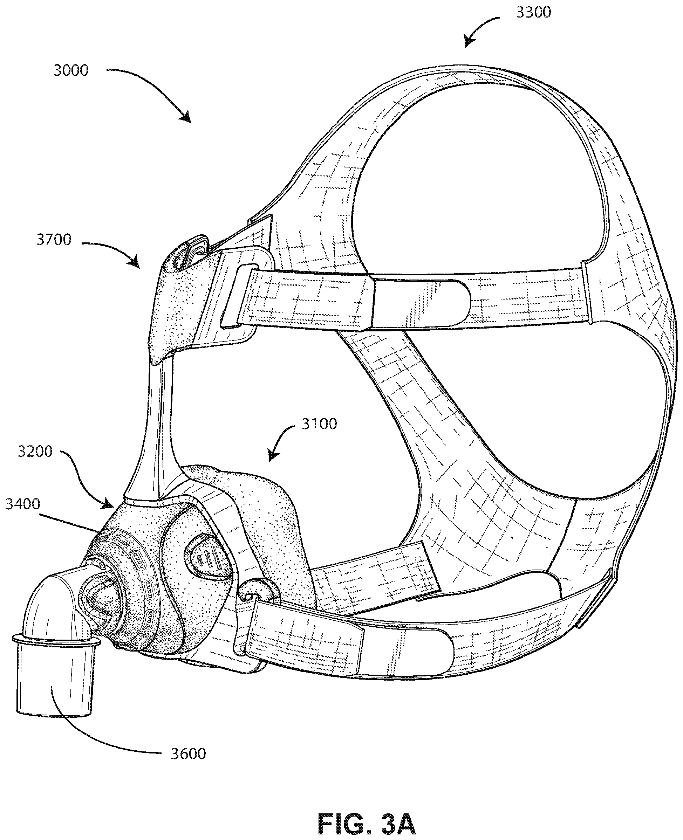

As shown in Fig. 3A, a non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

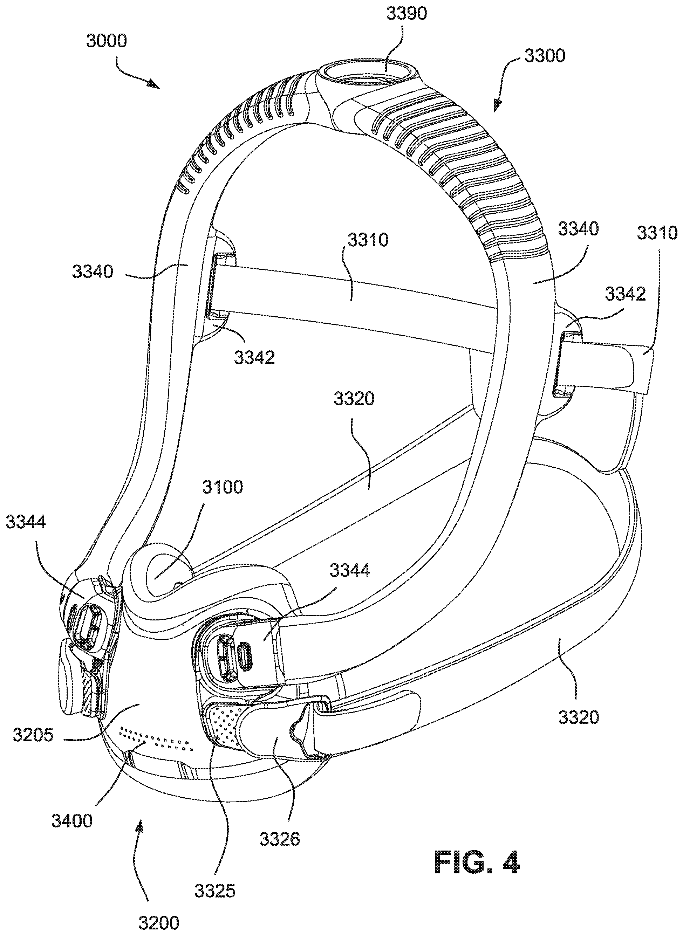

Fig. 4 shows a patient interface 3000 in accordance with another form of the present technology. The patient interface 3000 in this example also comprises a positioning and stabilising structure 3300 to hold the plenum chamber 3200 in sealing position on the patient's face in use. The positioning and stabilising structure 3300 in this example comprises a pair of headgear tubes 3340. The pair of headgear tubes 3340 are connected to each other at their superior ends and are each configured to lie against superior and lateral surfaces of the patient's head in use. Each of the headgear tubes 3340 may be configured to lie between an eye and an ear of the patient in use. The inferior end of each headgear tube 3340 is configured to fluidly connect to the plenum chamber 3200. In this example, the inferior end of each headgear tube 3340 connects to a headgear tube connector 3344 configured to connect to the shell 3205 of the plenum chamber 3200. The positioning and stabilising structure 3300 comprises a conduit headgear inlet 3390 at the junction of the two headgear tubes 3340. The conduit headgear inlet 3390 is configured to receive a pressurised flow of gas, for example via an elbow comprising a connection port 3600, and allow the flow of gas into hollow interiors of the headgear tubes 3340. The headgear tubes 3340 supply the pressurised flow of gas to the plenum chamber 3200.

The positioning and stabilising structure 3300 may comprise one or more straps in addition to the headgear tubes 3340. In this example the positioning and stabilising structure 3300 comprises a pair of upper straps 3310 and a pair of lower straps 3320. The posterior ends of the upper straps 3310 and lower straps 3320 are joined together. The junction between the upper straps 3310 and lower strap 3320 is configured to lie against a posterior surface of the patient's head in use, providing an anchor for the upper strap 3310 and lower straps 3320. Anterior ends of the upper straps 3310 connect to the headgear tubes 3340. In this example each headgear tube 3340 comprises a tab 3342 having an opening through which a respective upper strap 3310 can be passed through and then looped back and secured onto itself to secure the upper headgear strap 3310 to the headgear tube 3340. The positioning and stabilising structure 3300 also comprises a lower strap clip 3326 provided to the anterior end of each of the lower straps 3320. Each of the lower strap clip 3326 is configured to connect to a lower connection point 3325 on the plenum chamber 3200. In this example, the lower strap clips 3326 are secured magnetically to the lower connection points 3325. In some examples, there is also a mechanical engagement between the lower strap clips 3326 and the lower connection points 3325.

In some examples of the present technology, the plenum chamber 3200 is at least partially formed by the shell 3205 and the seal-forming structure 3100. The plenum chamber 3200 may comprise a cushion module or cushion assembly, for example. The shell 3205 may function as a chassis to support the seal-forming structure 3100.

The exemplary patient interface 3000 in Fig. 4 is an oronasal patient interface. That is, the patient interface 3000 is configured to seal around both the patient's nasal airways and oral airway. In some examples the patient interface 3000 comprises separate seals around each of the nasal airways and oral airway. The patient interface 3000 may comprise a plenum chamber 3200 having a nasal portion and an oral portion. The seal forming structure may be configured to surround the nasal airways at the nasal portion and to seal around the patient's mouth at the oral portion. As such, the seal-forming structure 3100 may also be considered to have a nasal portion and an oral portion, the nasal portions and oral portions of the seal-forming structure comprising those parts that seal around the patient's nasal airways and mouth respectively.

In an example, the seal-forming structure 3100 at the nasal portion does not lie over a nose bridge region or nose ridge region of the patient's face and instead seals against inferior surfaces of the patient's nose. The nasal portion may seal against the lip superior, the ala and the anterior surface of the pronasale and/or the inferior surface of the pronasale. The actual sealing locations may differ between patients. The nasal portion may also be configured to contact and/or seal to a region of the patient's face between the ala and the nasolabial sulcus and at the lateral portions of the lip superior proximate the nasolabial sulcus.

The seal-forming structure 3100 of the oral portion may be configured to form a seal to a periphery of the patient's mouth in use. The oral portion may be configured to form a seal to the patient's face at the lip superior, nasolabial sulcus, cheeks, lip inferior, supramenton, for example.

The seal-forming structure 3100 may have one or more holes therein such that the flow of air at a therapeutic pressure is delivered to the patient's nares and to the patient's mouth via the one or more holes. The seal-forming structure may define an oral hole and one or more nasal holes to deliver the flow of air to the patient. In an example, the plenum chamber 3200 comprises a seal-forming structure 3100 comprising an oral hole and two nasal holes. Each of the nasal holes may be positioned on the plenum chamber 3200 to be substantially aligned with a nare of the patient in order to deliver a flow of air thereto in use.

Further examples and details of the oronasal patient interface of Fig. 4 are described in PCT Application No. PCT/AU2019/050278, filed March 28, 2019.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH₂O with respect to ambient.

The patient interface 3000 in accordance with one form of the present technology is constructed and arranged to be able to provide a supply of air at a positive pressure of at least 10 cmH₂O with respect to ambient.

The patient interface 3000 in accordance with one form of the present technology is constructed and arranged to be able to provide a supply of air at a positive pressure of at least 20 cmH₂O with respect to ambient.

### 5.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 5.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 5.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 5.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 5.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 5.3.1.5 Forehead region

In one form, the seal-forming structure that forms a seal in use on a forehead region of the patient's face. In such a form, the plenum chamber may cover the eyes in use.

### 5.3.1.6 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 5.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

### 5.3.3 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

### 5.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO₂ by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

### Vent and AAV Arrangement

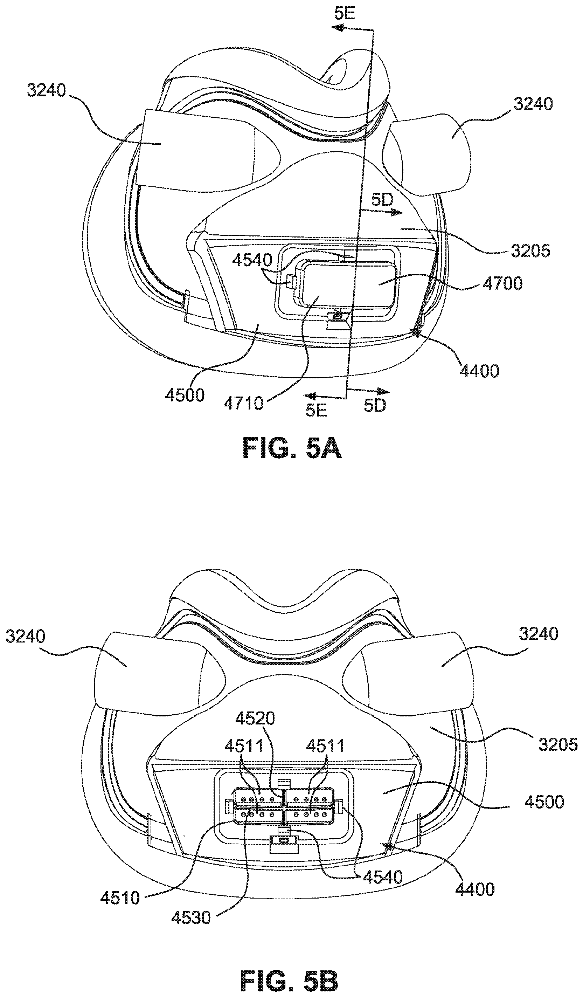

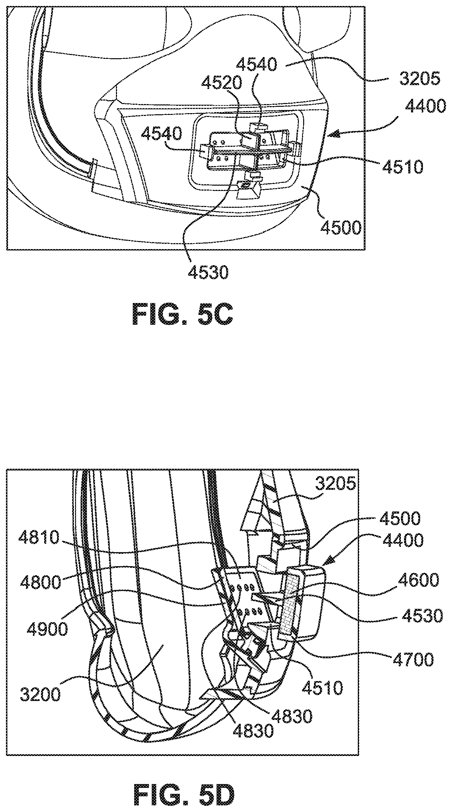

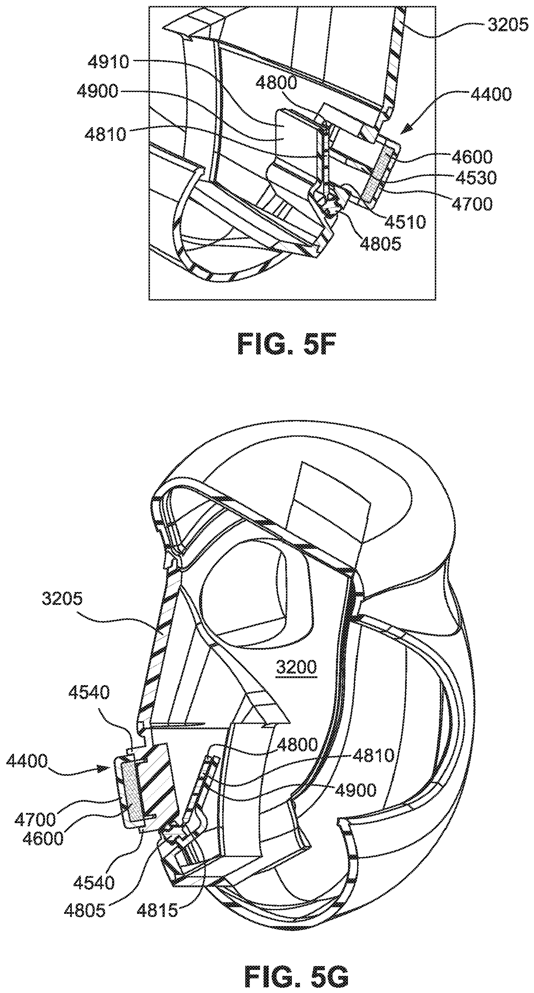

Figs. 5A to 5K show a vent and anti-asphyxia valve (AAV) arrangement 4400 for a patient interface according to an example of the present technology.

The vent and AAV arrangement 4400 is configured to provide a vent flow of gas to discharge gas exhaled by the patient. In the illustrated example, the vent and AAV arrangement 4400 comprises a diffusing member 4600 along the vent flow path structured and arranged to diffuse the exhaust vent flow to produce less noise. In addition, the vent and AAV arrangement 4400 is configured to allow the patient to breathe in ambient air and exhale through one or more openings if pressurized gas is not of sufficient magnitude or not delivered.

In the illustrated example, the patient interface 3000 is an oronasal patient interface, e.g., such as the type shown in Fig. 4. However, it should be appreciated that aspects of the present technology may be adapted for use with other suitable interface types.

In an illustrated example, the shell or chassis 3205 of the oronasal patient interface comprises two inlet ports 3240 provided to lateral sides of the shell 3205 (see Figs. 5A and 5B). The inlet ports 3240 in this example are configured to connect to respective ones of the headgear tubes 3340 to allow a pressurized flow of gas to pass through the hollow interiors of the headgear tubes 3340, through respective inlet ports 3240, and into the plenum chamber 3200. In an example, the inlet ports 3240 may receive combined headgear and conduit connection assemblies (e.g., headgear tube connector 3344 as shown in Fig. 4) in order to provide multiple functions such as supply of the flow of air and headgear attachment points.

In the illustrated example, a port or opening 4510 is provided to the shell 3205 inferior to the inlet ports 3240. The port 4510 is configured to allow gas to flow between the plenum chamber and ambient. As described below, the anti-asphyxia valve (AAV) member 4800 of the vent and AAV arrangement 4400 is configured and arranged to deflect or pivot to regulate air flow through the port 4510.

In the illustrated example, the port 4510 is provided centrally with respect to the plenum chamber 3200, e.g., so that the vent and AAV arrangement 4400 is located where it is most effective for reducing CO₂, i.e., aligned approximately with the patient's mouth which provides a large portion of inhaled/exhaled gas. Also, such positioning of the port 4510 is less prone to being blocked during side sleeping.

Furthermore, since the inlet ports 3240 of the plenum chamber 3200 are provided at a superior location on the plenum chamber 3200, a bias flow of air received at the inlet ports 3240 may flow through a large volume (e.g., from a superior location to an inferior location), which may provide for efficient gas washout and may reduce the likelihood of stagnant air pockets bypassed by the bias flow.

In use, the vent and AAV arrangement 4400 is structured and arranged to allow gas flow between an interior of the patient interface 3000, e.g., the plenum chamber 3200, and an exterior of the patient interface 3000, e.g., atmosphere.

In the illustrated example, e.g., see Figs. 5A to 5G, the vent and AAV arrangement 4400 comprises a shell portion 4500 that surrounds and forms the port 4510, a diffusing member 4600 provided to an anterior side of the shell portion 4500, a diffusing member cover 4700 to maintain the diffusing member 4600 to the shell portion 4500, an AAV member 4800 provided to a posterior side of the shell portion 4500, and an AAV guard 4900 to define a stop for the AAV member 4800. In use, the AAV member 4800 is structured and arranged to regulate air flow through the port 4510 and to provide sufficient washout of gas in use.

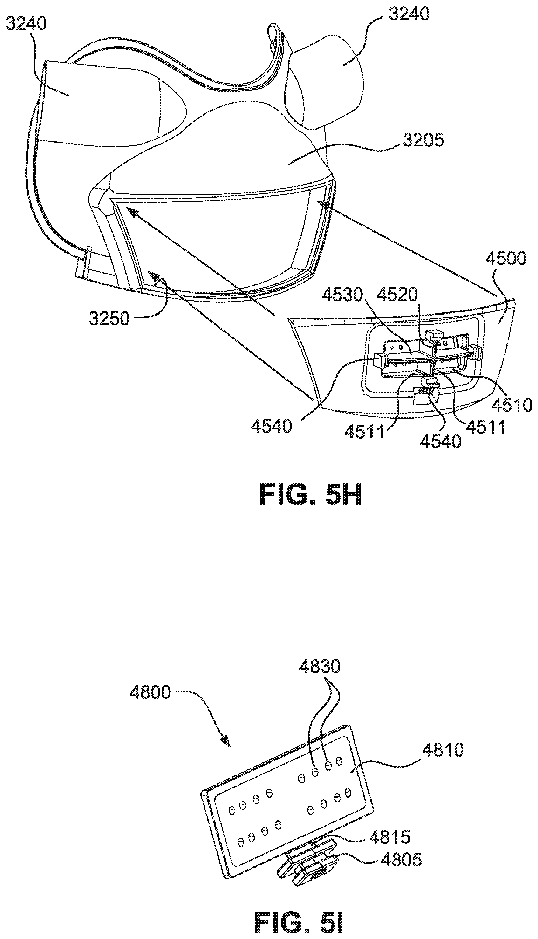

In the illustrated example, the shell portion 4500 is in the form of an insert structured to be inserted into an opening 3250 in the shell 3205 (e.g., see Fig. 5H). The shell portion 4500 may be removably or permanently secured within the opening 3250 in any suitable manner, e.g., press fit assembly, snap or interference fit assembly, adhesive. In an alternative example, the shell portion 4500 may be formed in one-piece with the shell 3205, e.g., by over-molding or insert-molding. In another example, the shell portion 4500 and port 4510 thereof may be simply integrated into the shell 3205 and form an integral, one-piece molded construction.

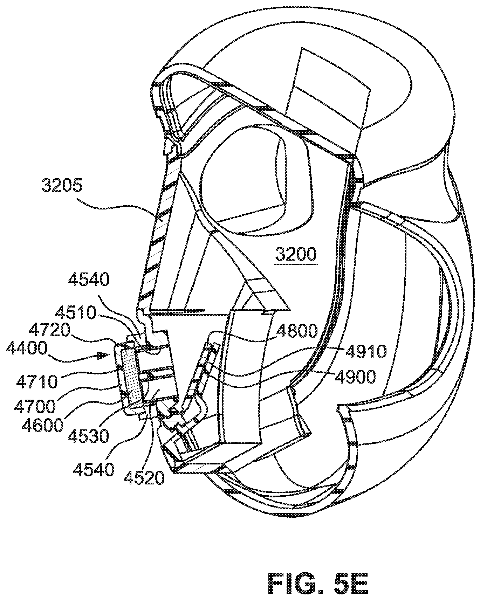

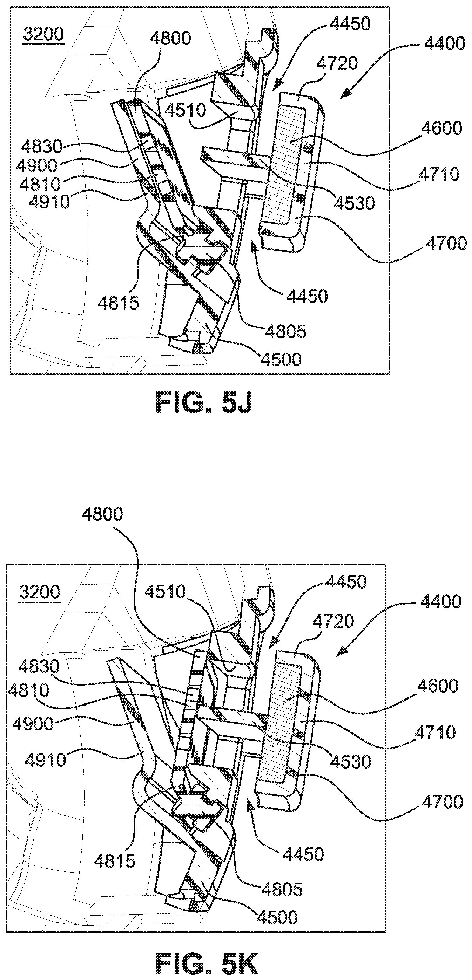

In the illustrated example, the port 4510 in the shell portion 4500 includes a generally rectangular shape, however it should be appreciated that other suitable shapes are possible, e.g., circular and non-circular shapes. A vertical support 4520 extends along the minor axis across the port 4510, and a horizontal support 4530 extends along the major axis across the port 4510. The supports 4520, 4530 form a grating to support the diffusing member 4600 along the anterior side of the port 4510 and to prevent the diffusing member 4600 from passing or extending through the port 4510 into the plenum chamber 3200 (e.g., see Figs. 5D, 5E, 5F, and 5K). Further, the supports 4520, 4530 form a grating to define a stop for the AAV member 4800 and to prevent the AAV member 4800 from passing through or getting lodged in the port 4510 (e.g., see Fig. 5K). As illustrated, e.g., see Figs. 5B and 5H, the supports 4520, 4530 divide the port 4510 into a plurality of port openings 4511.

The diffusing member 4600 is supported along the anterior side of the shell portion 4500 by the supports 4520, 4530 extending across the port 4510, so that the diffusing member 4600 is arranged to cover the anterior end of the port 4510 and at least a portion of the flow exiting the port 4510 (e.g., vent flow) can flow into the diffusing member 4600. As illustrated, the supports 4520, 4530 are structured and arranged to protrude beyond an anterior side of the shell portion 4500, so that the diffusing member 4600 is offset or supported in spaced relation from the anterior end of the port 4510. This offset allows at least a portion of the flow exiting and/or entering the port 4510 to bypass the diffusing member 4600 and flow through lateral openings 4450 (e.g., see Figs. 5K and 5J) formed between the diffusing member 4600 and shell portion 4500.

In an example, the diffusing member 4600 may be constructed of a porous material that allows gas to flow through the material but diffuses any jet or other flow formation entering and/or exiting the port 4510, e.g., textile material such as a nonwoven fibrous material or a woven fibrous material. In an example, the diffusing member 4600 may comprise a diffusing material which may be similar to or the same as a filter media. In the illustrated example, the diffusing member 4600 comprises a single layer, however it should be appreciated that the diffusing member 4600 may comprise two or more layers, e.g., stacked layers, of similar or dissimilar diffusing materials.

The diffusing member cover 4700 includes an anterior wall 4710 and a side wall 4720 extending along a perimeter of the anterior wall 4710. The anterior wall 4710 and the side wall 4720 form a cavity to receive the diffusing member 4600.

The diffusing member cover 4700 is engaged with the shell portion 4500 to secure the cover 4700 to the shell portion 4500 and support and retain the diffusing member 4600 to the shell portion 4500. In the illustrated example, the shell portion 4500 includes support members 4540 along the outer periphery of the port 4510 to support and retain the cover 4700, e.g., support members 4540 at respective ends of the vertical support 4520 and support members 4540 at respective ends of the horizontal support 4530. The support members 4540 are structured and arranged to protrude beyond an anterior side of the shell portion 4500, and forms a raised platform to abut the side wall 4720 and support the cover 4700 (and the diffusing member 4600) in spaced relation from the outlet end of the port 4510, e.g., to minimize noise. Also, such spacing or offset of the cover 4700 and the diffusing member 4600 from the outlet end of the port 4510 forms the lateral openings 4450, which ensures that the port 4510 does not become occluded by the diffusing member 4600 at any time.

The cover 4700 may be removably or permanently secured to the support members 4540 of the shell portion 4500 in any suitable manner, e.g., press fit assembly, magnetic assembly, snap or interference fit assembly, adhesive, ultrasonic welding, etc. In an example, the cover 4700 may be removably secured to the shell portion 4500, e.g., to allow cleaning and/or replacement of the diffusing member 4600.

In the illustrated example, the anterior wall 4710 of the cover 4700 is devoid of any openings, e.g., so that at least a portion of the flow exiting the port 4510 (e.g., vent flow) can flow into the diffusing member 4600 and then be redirected laterally to flow through the lateral openings 4450 to atmosphere. That is, the anterior wall 4710 completely covers an anterior side of the diffusing member and prevents flow from passing through the anterior side of the diffusing member 4600, and then through the cover 4700 to ambient. In an alternative example, the anterior wall 4710 of the cover 4700 may include one or more openings so that at least a portion of the flow exiting the port 4510 can flow through the anterior side of the diffusing member 4600 and through the openings of the cover 4700 to ambient.

The AAV member 4800, e.g., see Fig. 5I, includes a single flap arrangement structured and arranged to selectively cover the port 4510 in use. As illustrated, the AAV member 4800 includes a retaining portion 4805, a flap portion 4810 that is movably connected, e.g., hingedly connected by a hinge portion 4815, to the retaining portion 4805 which allows the flap portion 4810 to pivot relative to the retaining portion 4805. The flap portion 4810 includes a plurality of vent holes 4830 extending therethrough. In the illustrated example, the plurality of vent holes 4830 are arranged in rows (e.g., 4 rows of 4 holes), however the vent holes 4830 may be arranged in other suitable manners. In the illustrated example, e.g., see Fig. 5B, each of the rows of the vent holes 4830 is arranged to align with a corresponding one of the port openings 4511 of port 4510 formed by the supports 4530, 4530.

As best shown in Figs. 5D to 5G and 5J to 5K, the retaining portion 4805 of the AAV member 4800 is engaged with an opening provided to the shell portion 4500 to retain the AAV member 4800 to the shell portion 4500. The retaining portion 4805 may be removably or permanently secured to the shell portion 4500 in any suitable manner, e.g., press fit assembly, snap or interference fit assembly, adhesive, ultrasonic welding, etc. In an example, the retaining portion 4805 may be removably secured to the shell portion 4500, e.g., to allow cleaning and/or replacement of the AAV member 4800.

The AAV guard 4900 includes a wall 4910 oriented at an angle to the shell portion 4500, i.e., wall 4910 extends at an angle from the shell portion 4500 into the plenum chamber 3200. In the illustrated example, the wall 4910 forms an acute angle with respect to the shell portion 4500, e.g., 20°-60°, e.g., 30°-45°. The acute angle may be adjusted so that there is smoother fluid flow. The wall 4910 may be a solid wall (i.e., the wall 4910 does not have any holes therethrough) or the wall 4910 may have one or more holes therethrough. Preferably, the wall 4910 does not have any holes to prevent the flap portion from closing (e.g., during patient exhalation). In the illustrated example, the AAV guard 4900 is integrated with the shell portion 4500 to form an integral, one-piece molded construction. In an alternative example, the AAV guard 4900 may be provided as a separate structure from the shell portion 4500 that is attached or otherwise secured to the shell portion 4500.

In this example, the vent and AAV arrangement 4400 includes a single port 4510 that is closable by a single flap portion 4810 of the AAV member 4800. As such, the size of the port 4510 (and hence the flap portion 4810) is sufficiently large to provide sufficient AAV performance (e.g., due to deadspace in the headgear tubes 3340). In addition, the working angle or hinge opening radius for the flap portion (e.g., defined by the AAV guard 4900) is sufficiently large (e.g., 30°-45°) to provide the required opening to the port 4510 for sufficient AAV performance.

In the illustrated example, as shown in Fig. 5J, the flap portion 4810 of the AAV member 4800 is biased or pre-loaded relative to the retaining portion 4805 into engagement with the wall 4910 of the AAV guard 4900 which defines a stop for the flap portion 4810. This arrangement allows the flap portion 4810 to remain in a "rest" position when pressurized gas is not of sufficient magnitude or not delivered. In the illustrated example, the wall 4910 is structured to cover or engage along the entirety of the flap portion 4810. However, it should be appreciated that the wall 4910 may be modified to cover or engage along a smaller portion of the flap portion 4810. Also, the AAV guard 4900 prevents flow (e.g., from patient exhalation) from deactivating or closing the flap portion 4810, and prevents the flap portion 4810 from significant protrusion into the plenum chamber 3200 when activated.

The AAV member 4800 is supported adjacent the posterior side of the shell portion 4500, and the flap portion 4810 is movable towards and away from the port 4510, e.g., depending on the presence of pressurized gas.

The plurality of vent holes 4830 of the flap portion 4810 are arranged to align with respective port openings 4511 of port 4510 so that the port 4510 cannot be completely covered or closed by the flap portion 4810 to allow flow through the port 4510.

In an example, the AAV member 4800 may comprise a one-piece construction of a relatively flexible, elastic material, e.g., silicone or other thermoplastic elastomer. In another example, the AAV member 4800 may comprise a one-piece construction of a plastic material, e.g., polycarbonate. In another example, the AAV member 4800 may comprise a combination of elastic and plastic materials, e.g., flap portion 4810 comprising a plastic material (e.g., polycarbonate) and the retaining portion 4805 and hinge portion 4815 comprising an elastic material (e.g., silicone). In each example, the plurality of vent holes 4830 in the flap portion 4810 may be molded or laser cut. In an example, the flap portion 4810 comprising a plastic material (e.g., polycarbonate) may provide better tolerances for the plurality of vent holes 4830.

In an alternative example, the gas washout vent provided to the flap portion 4810 may be a textile vent (e.g., one or a plurality of vent holes formed by interspaces between the fibers of a textile material) or a microvent (e.g., plurality of micro-sized vent holes (diameter of 1 micron or less) formed in a substrate of a semipermeable material (e.g., using a laser drill or chemical etchant)). In an example, the textile vent or microvent may provide about 20-80 vent holes.

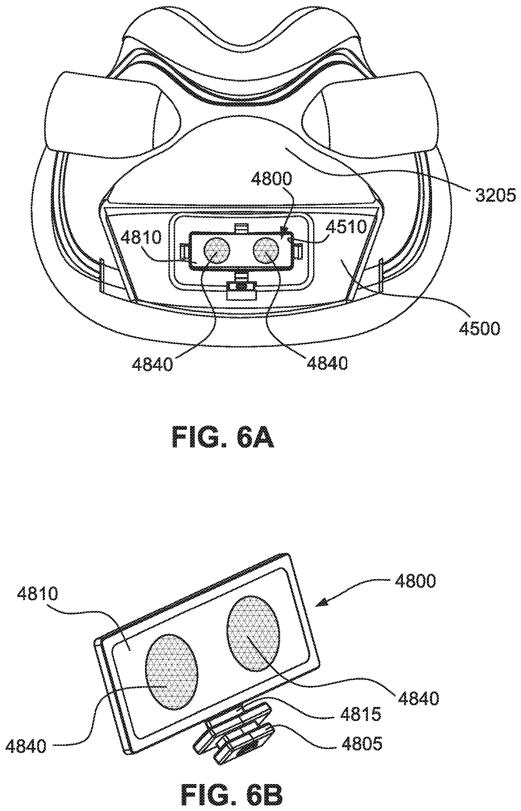

For example, Figs. 6A and 6B shows an AAV member 4800 with two textile or microvent vent portions 4840 provided to the flap portion 4810. In the illustrated example, each of the vent portions 4840 includes a circular configuration. However, it should be appreciated that each vent portion 4840 may include other suitable shapes (e.g., non-circular shapes) and the vent portions 4840 may be arranged on the flap portion 4810 in other suitable manners. In addition, it should be appreciated that the number of vent portions (e.g., one or more vent portions 4840 on the flap portion 4810), size of each vent portion (e.g., area of each vent portion), and vent porosity (e.g., vent hole size) provided to each vent portion may be modified, e.g., depending on the desired gas washout rate.

In an example, the textile or microvent vent portions 4840 and the flap portion 4810 may comprise an overmolded construction, i.e., elastic or plastic flap portion 4810 overmolded to the vent portions 4840. However, the vent portions 4840 may be provided to the flap portion 4810 in other suitable manners, e.g., vent portions 4840 adhered or otherwise permanently secured to the flap portion 4810 in an operative position.

In the illustrated example, e.g., see Fig. 6A, the vent and AAV arrangement may be provided without a diffusing member 4600 and diffusing member cover 4700, i.e., textile or microvent vent portions 4840 may provide sufficient diffusivity (e.g., better than multiple vent holes 4830) to reduce noise.

Further examples and details of textile vents or microvents are described in PCT Publication No. WO2014/015382.

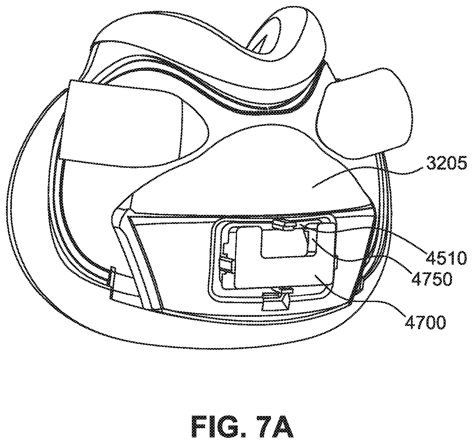

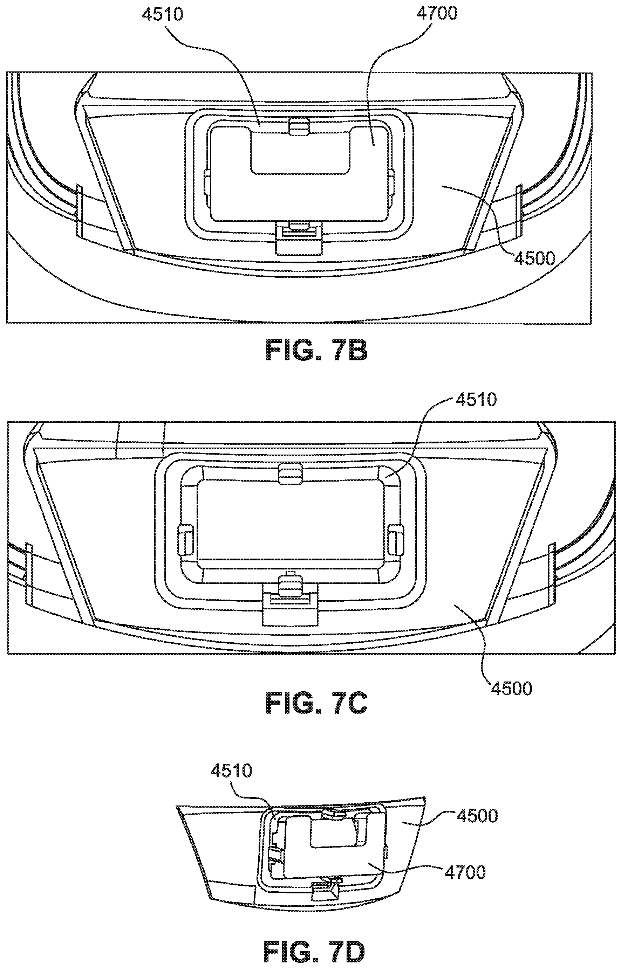

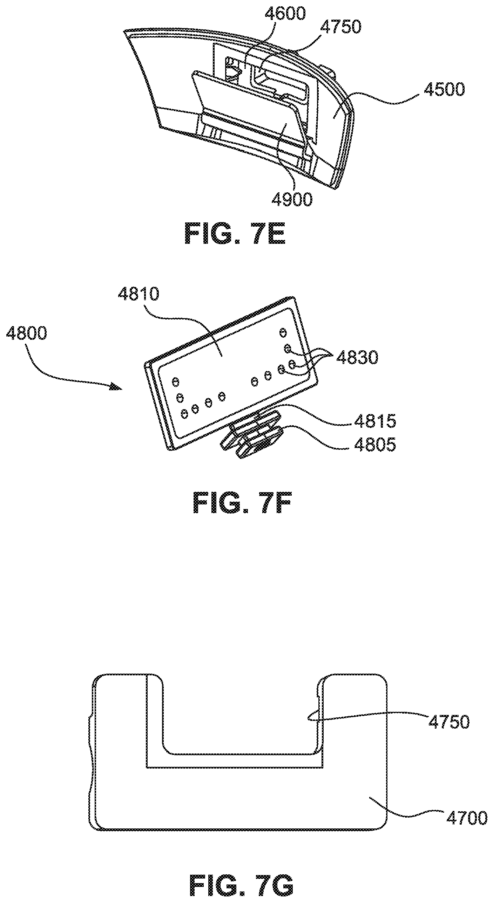

As noted above, the vent holes 4830 on the flap portion 4810 may be arranged in other suitable manners. For example, in an alternative example as shown in Figs. 7A to 7G, the vent holes 4830 may be arranged in a general U-shape on the flap portion 4810, i.e., in a row along a bottom side of the flap portion 4810 and in columns along respective lateral sides of the flap portion 4810 (see Fig. 7F). In the illustrated example, the diffusing member 4600 and diffusing member cover 4700 includes a corresponding shape to the vent hole arrangement, e.g., general U-shape. As such, the diffusing member 4600 and diffusing member cover 4700 are arranged to only cover the vent holes 4830 on the flap portion 4810 so that at least a portion of the vent flow exiting the vent holes can flow into the diffusing member 4600. Moreover, the diffusing member 4600 and diffusing member cover 4700 forms an opening 4750 that aligns with the port 4510 so that at least a portion of the port 4510 is not covered and at least a portion of flow entering and/or exiting the port 4510 will not flow into the diffusing member 4600. As shown in Fig. 7C, in this example, the port 4510 is devoid of any supports extending across the port 4510.

In an example, the shell portion 4500, cover 4700, and AAV guard 4900 may be constructed (e.g., molded) of a relatively rigid material, e.g., thermoplastic polymer (e.g., polycarbonate). In an example, the shell portion 4500 and AAV guard 4900 may be formed in one piece with the shell 3205, e.g., by over-molding or insert-molding.

The vent and AAV arrangement 4400 is structured and arranged to regulate flow therethrough to (1) provide a vent flow path when pressure in the patient interface is above a predetermined magnitude and (2) provide a breathable flow path when pressure in the patient interface is below a predetermined magnitude or not delivered.

As shown in Fig. 5J, when pressure in the patient interface is below a predetermined magnitude or not delivered (e.g., when the RPT device is not operating), the flap portion 4810 of the AAV member 4800 assumes an activated position to uncover or open the port so that air may pass through the port 4510. That is, pressurized gas is not delivered to the patient interface or is not of sufficient magnitude which allows the flap portion 4810 to be biased or deflected downwardly away from the shell portion 4500 and the port 4510 and into engagement with the wall 4910 of the AAV guard 4900. As a result, air may pass through breathable flow paths that extend through the port openings 4511 of the port 4510, and through the lateral openings 4450 between the cover 4700/diffusing member 4600 and the shell portion 4500. Such breathable flow paths provide air paths to allow the patient to breathe in ambient air and exhale. It is noted that air may potentially flow through the diffusing member 4600, but will have low impact on AAV performance.

As shown in Fig. 5K, when pressure in the patient interface is above a predetermined magnitude (e.g., when the RPT device is operating), the increase in pressure within the pressurized volume of the patient interface creates a pressure gradient or vacuum (flow through the port 4510 creates negative pressure) which draws or sucks the flap portion 4810 into a deactivated position to cover or close the port 4510 so that air may only pass through the plurality of vent holes 4830 in the flap portion 4810 and through the port openings 4511 of the shell portion 4500. The pressure in the patient interface is of sufficient magnitude to force and maintain the flap portion 4810 in engagement with the posterior side of the shell portion 4500 and the supports 4520, 4530. As a result, the plurality of vent holes 4830 in the flap portion 4810 are arranged to align with respective port openings 4511 so that air may pass through vent flow paths that extend through the vent holes 4830 of the flap portion 4810, through the port openings 4511 of the shell portion 4500, into the diffusing member 4600, and then redirected by the cover 4700 through the lateral openings 4450 between the cover 4700/diffusing member 4600 and the shell portion 4500. Also, some air may bypass the diffusing member 4600 and may flow through the vent holes 4830, through the port openings 4511, and through the lateral openings 4450. Such vent flow paths allow sufficient gas washout to prevent CO₂ build-up in the patient interface. Vent flow through the diffusing member 4600 may diffuse the exhaust vent flow to produce less noise. Vent flow that bypasses the diffusing member 4600 may provide an alternative path to allow sufficient gas washout when the diffusing member 4600 is blocked due to water or humidity.

Aspects of the vent and AAV arrangement 4400 may be tuned to provide a desired flow curve within a therapeutic pressure range. In an example, venting characteristics of each of the components may be tuned, e.g., based on venting requirement, sound requirement, treatment requirement, etc. This arrangement allows a more customized vent and AAV arrangement for the patient. For example, the shape, size, and number of vent holes 4830 through the flap portion 4810 may be tuned to regulate vent flow.

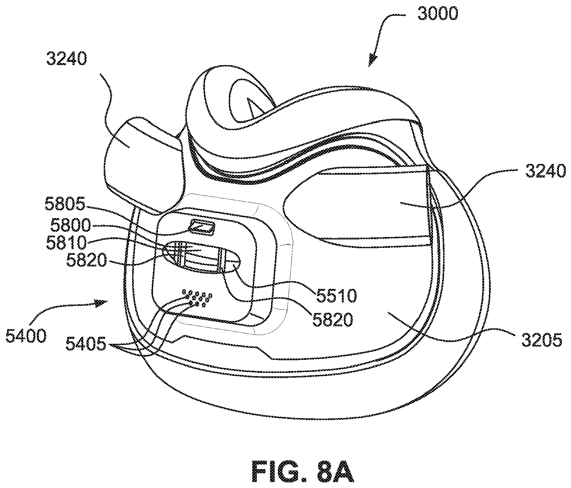

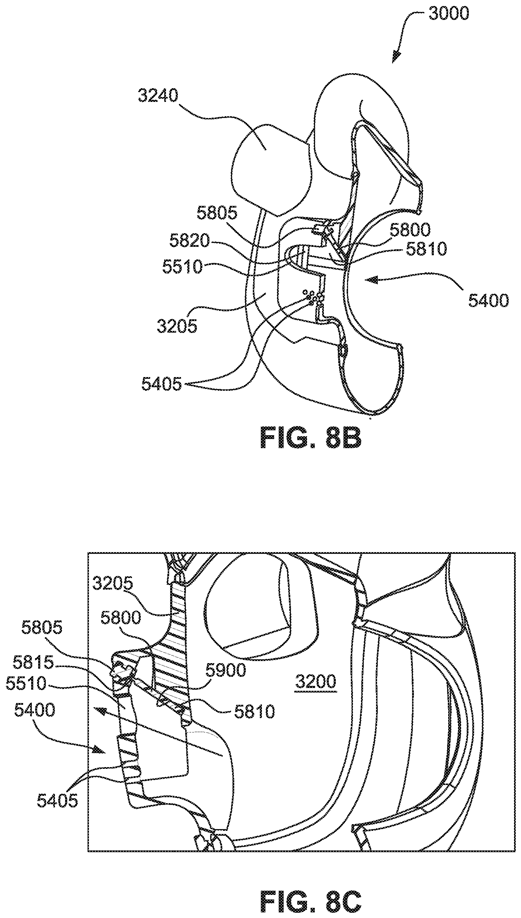

Figs. 8A to 8C show a vent and AAV arrangement 5400 for a patient interface according to another example of the present technology. In this example, vent holes 5405 are provided to the shell 3205 of the patient interface 3000, rather than the AAV member 5800.

In the illustrated example, a port or opening 5510 is provided to the shell 3205 inferior to the inlet ports 3240. The AAV member 5800 is supported by the shell 3205 superior the port 5510, and is configured and arranged to deflect or pivot to regulate air flow through the port 5510.

In the illustrated example, the port 5510 (and the flap portion 5810 of the AAV member 5800) includes a generally oval shape, however it should be appreciated that other suitable shapes are possible, e.g., circular and non-circular shapes. Vertical supports 5520 extend across the port 5510 to form a grating to define a stop for the AAV member 5800 and prevent the AAV member 5800 from passing through or getting lodged in the port 5510.

The AAV member 5800 includes a single flap arrangement structured and arranged to selectively cover the port 5510 in use. As illustrated, the AAV member 5800 includes a retaining portion 5805, a flap portion 5810 that is movably connected, e.g., hingedly connected by a hinge portion 5815, to the retaining portion 5805 which allows the flap portion 5810 to pivot relative to the retaining portion 5805.

The retaining portion 5805 of the AAV member 5800 is engaged with an opening provided to the shell 3205 (superior the port 5510) to retain the AAV member 5800 to the shell 3205. The retaining portion 5805 may be removably or permanently secured to the shell 3205 in any suitable manner, e.g., press fit assembly, snap or interference fit assembly, adhesive, ultrasonic welding, etc. In an example, the retaining portion 5805 may be removably secured to the shell 3205, e.g., to allow cleaning and/or replacement of the AAV member 5800.

The shell 3205 includes an interior wall that provides an AAV guard or stop surface 5900 oriented at an angle to the front wall of shell 3205 in which the port 5510 is provided. In the illustrated example, the AAV guard or stop surface 5900 forms an acute angle with respect to the front wall of the shell 3205, e.g., 20°-60°, e.g., 30°-45°.

In the illustrated example, the flap portion 5810 of the AAV member 5800 is biased or pre-loaded relative to the retaining portion 5805 into engagement with the AAV guard or stop surface 5900 which defines a stop for the flap portion 5810. This arrangement allows the flap portion 5810 to remain in a "rest" position when pressurized gas is not of sufficient magnitude or not delivered. In the illustrated example, the AAV guard or stop surface 5900 is arranged to prevent flow (e.g., from patient exhalation) from deactivating or closing the flap portion 5810, and prevents the flap portion 5810 from significant protrusion into the plenum chamber 3200 when activated.

The AAV member 5800 is supported adjacent the posterior side of the shell 3205, and the flap portion 5810 is movable towards and away from the port 5510, e.g., depending on the presence of pressurized gas.

As shown in Fig. 8C, when pressure in the patient interface is below a predetermined magnitude or not delivered (e.g., when the RPT device is not operating), the flap portion 5810 of the AAV member 5800 assumes an activated position so that air may pass through the port 5510. That is, pressurized gas is not delivered to the patient interface or is not of sufficient magnitude which allows the flap portion 5810 to be biased or deflected away from the port 5510 and into engagement with the AAV guard or stop surface 5900. As a result, air may pass through a breathable flow path that extends through the port 5510. Such breathable flow path provides an air path to allow the patient to breathe in ambient air and exhale.

When pressure in the patient interface is above a predetermined magnitude (e.g., when the RPT device is operating), the increase in pressure within the pressurized volume of the patient interface creates a pressure gradient or vacuum (flow through the port 5510 creates negative pressure) which draws or sucks the flap portion 5810 into a deactivated position so that air cannot pass through the port 5510. The pressure in the patient interface is of sufficient magnitude to force and maintain the flap portion 5810 in engagement with the posterior side of the shell 3205 and the supports 5520.

In this example, the shell 3205 includes a plurality of vent holes 5405 extending therethrough, which allows sufficient gas washout to prevent CO₂ build-up in the patient interface. As illustrated, the vent holes 5405 are arranged inferior to and spaced apart from the port 5510, so that the vent holes 5405 cannot be covered or closed by the flap portion 5810 of the AAV member 5800 to allow vent flow through the vent holes 5405.

In the illustrated example, the plurality of vent holes 5405 are arranged in rows (e.g., 3 rows), however the vent holes 5405 may be arranged in other suitable manners. Also, the shape, size, and number of vent holes 5405 through the shell 3205 may be tuned to regulate vent flow.

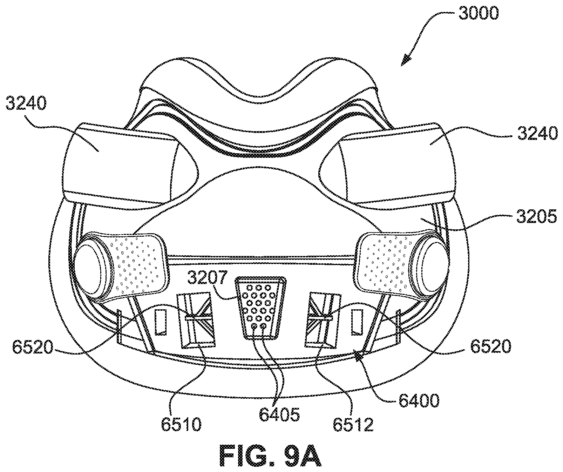

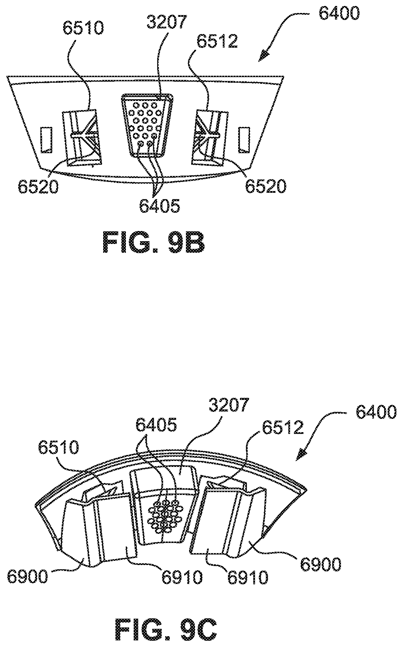

Figs. 9A to 9C show a vent and AAV arrangement 6400 for a patient interface according to another example of the present technology. Similar to the example of Figs. 8A to 8C, vent holes 6405 are provided to the shell 3205 of the patient interface 3000, rather than the AAV members 6800.

In this example, the shell 3205 includes dual ports 6510, 6512 (i.e., first and second spaced-apart ports) inferior to the inlet ports 3240, and dual AAV members (i.e., first and second AAV members) each configured and arranged to deflect or pivot to regulate air flow through respective ones of the ports 6510, 6512.

The shell 3205 includes a plurality of vent holes 6405 arranged between and spaced apart from the ports 6510, 6512 (so that the vent holes 6405 cannot be covered or closed by the flap portion of the AAV members), which allows sufficient gas washout to prevent CO₂ build-up in the patient interface. In the illustrated example, the plurality of vent holes 6405 are arranged in rows (e.g., 6 rows), however the vent holes 6405 may be arranged in other suitable manners. Also, the shape, size, and number of vent holes 6405 through the shell 3205 may be tuned to regulate vent flow. In the illustrated example, the vent holes 6405 are provided to a recessed portion 3207 of the shell 3205, e.g., to further enhance diffusivity of the vent flow.

In the illustrated example, each of the ports 6510, 6512 (and the flap portion of each of the AAV members) includes a generally rectangular shape, however it should be appreciated that other suitable shapes are possible, e.g., circular and non-circular shapes. Horizontal and angular supports 6520 extend across each of the ports 6510, 6512 to form a grating to define a stop for respective AAV members and prevent the AAV members from passing through or getting lodged in respective ports 6510, 6512.

An AAV guard 6900 is provided adjacent to each of the ports 6510, 6512 to define a stop for respective flap portions of the AAV members when in the activated position. The AAV guard 6900 includes a wall 6910 oriented at an angle from the shell 3205 into the plenum chamber, e.g., 20°-60°, e.g., 30°-45°.

Similar to the above examples, when pressure in the patient interface is below a predetermined magnitude or not delivered (e.g., when the RPT device is not operating), the flap portion of the AAV members assume an activated position (biased or deflected away from respective ports 6510, 6512 and into engagement with respective AAV guards 6900) so that air may pass through the ports 6510, 6512. When pressure in the patient interface is above a predetermined magnitude (e.g., when the RPT device is operating), the increase in pressure within the pressurized volume of the patient interface creates a pressure gradient or vacuum (flow through the ports 6510, 6512 creates negative pressure) which draws or sucks the flap portion of the AAV members into a deactivated position so that air cannot pass through the ports 6510, 6512.

Since the AAV arrangement includes dual ports 6510, 6512 with dual AAV members (rather than a single port with a single AAV member), the required opening for sufficient AAV performance may be split between the two ports/two AAV members, e.g., each port and corresponding opening may be smaller and/or the working angle for each flap portion may be less. This arrangement may provide smaller profile with AAV members/AAV guards that protrude less into the breathing chamber.

### 5.3.5 Decoupling structure(s)

In one form the patient interface 3000 includes at least one decoupling structure, for example, a swivel or a ball and socket.

### 5.3.6 Connection port

Connection port 3600 allows for connection to the air circuit 4170.

### 5.3.7 Forehead support

In one form, the patient interface 3000 includes a forehead support 3700.

### 5.3.8 Anti-asphyxia valve

In one form, the patient interface 3000 includes an anti-asphyxia valve.

### 5.3.9 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 5.4 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block of the RPT device 4000 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller. One example of an air circuit 4170 comprising a heated wire circuit is described in United States Patent 8,733,349.

### 5.4.1 Supplementary gas delivery

In one form of the present technology, supplementary gas, e.g. oxygen, may be delivered to one or more points in the pneumatic path, such as upstream of the pneumatic block, to the air circuit 4170, and/or to the patient interface 3000.

### 5.5 GLOSSARY

For the purposes of the present technology disclosure, in certain forms of the present technology, one or more of the following definitions may apply. In other forms of the present technology, alternative definitions may apply.

### 5.5.1 General

*Air*: In certain forms of the present technology, air may be taken to mean atmospheric air, and in other forms of the present technology air may be taken to mean some other combination of breathable gases, e.g. oxygen enriched air.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy*: CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy*: Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate*: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol *Q*. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Oxygen enriched air:* Air with a concentration of oxygen greater than that of atmospheric air (21%), for example at least about 50% oxygen, at least about 60% oxygen, at least about 70% oxygen, at least about 80% oxygen, at least about 90% oxygen, at least about 95% oxygen, at least about 98% oxygen, or at least about 99% oxygen. "Oxygen enriched air" is sometimes shortened to "oxygen".

*Medical Oxygen:* Medical oxygen is defined as oxygen enriched air with an oxygen concentration of 80% or greater.

*Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy:* The application of a supply of air to an entrance to the airways at a treatment pressure that is typically positive with respect to atmosphere.

*Ventilator:* A mechanical device that provides pressure support to a patient to perform some or all of the work of breathing.

### 5.5.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 5.5.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.5.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Ttot*.

*Effort (breathing):* The work done by a spontaneously breathing person attempting to breathe.

*Expiratory portion of a breathing cycle:* The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
*(i) Flattened:* Having a rise followed by a relatively flat portion, followed by a fall.
(ii) *M-shaped:* Having two local peaks, one at the leading edge, and one at the trailing edge, and a relatively flat portion between the two peaks.
(iii) *Chair-shaped:* Having a single local peak, the peak being at the leading edge, followed by a relatively flat portion.
(iv) *Reverse-chair shaped:* Having a relatively flat portion followed by single local peak, the peak being at the trailing edge.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea:* An increase in flow to a level higher than normal.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Peak flow rate (Qpeak):* The maximum value of flow rate during the inspiratory portion of the respiratory flow waveform.

*Respiratory flow rate,* patient *airflow rate, respiratory airflow rate (Qr):* These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt):* The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti):* The duration of the inspiratory portion of the respiratory flow rate waveform.

*(exhalation) Time (Te):* The duration of the expiratory portion of the respiratory flow rate waveform.

*(total) Time (Ttot):* The total duration between the start of one inspiratory portion of a respiratory flow rate waveform and the start of the following inspiratory portion of the respiratory flow rate waveform.

*Typical recent ventilation:* The value of ventilation around which recent values of ventilation *Vent* over some predetermined timescale tend to cluster, that is, a measure of the central tendency of the recent values of ventilation.

*Upper airway obstruction (UAO):* includes both partial and total upper airway obstruction. This may be associated with a state of flow limitation, in which the flow rate increases only slightly or may even decrease as the pressure difference across the upper airway increases (Starling resistor behaviour).

*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.5.3 Anatomy

### 5.5.3.1 Anatomy of the face

*Ala:* the external outer wall or "wing" of each nostril (plural: alar).

*Alare:* The most lateral point on the nasal *ala.*

*Alar curvature (or alar crest) point:* The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework:* The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane:* A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

*Greater alar cartilage:* A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils):* Approximately ellipsoidal apertures forming the entrance to the nasal cavity. The singular form of nares is naris (nostril). The nares are separated by the nasal septum.

*Naso-labial sulcus or Naso-labial fold:* The skin fold or groove that runs from each side of the nose to the corners of the mouth, separating the cheeks from the upper lip.

*Naso-labial angle:* The angle between the columella and the upper lip, while intersecting subnasale.

*Otobasion inferior:* The lowest point of attachment of the auricle to the skin of the face.

*Otobasion superior:* The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal):* The nasal ridge is the midline prominence of the nose, extending from the Sellion to the Pronasale.

*Sagittal plane:* A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare:* The point at the lower margin of the alar base, where the alar base joins with the skin of the superior (upper) lip.

*Subnasal point:* Located on the soft tissue, the point at which the columella merges with the upper lip in the midsagittal plane.

*Supramenton:* The point of greatest concavity in the midline of the lower lip between labrale inferius and soft tissue pogonion.

### 5.5.3.2 Anatomy of the skull

*Frontal bone:* The frontal bone includes a large vertical portion, the *squama frontalis,* corresponding to the region known as the forehead.

*Mandible:* The mandible forms the lower jaw. The mental protuberance is the bony protuberance of the jaw that forms the chin.

*Maxilla:* The maxilla forms the upper jaw and is located above the mandible and below the orbits. The *frontal process of the maxilla* projects upwards by the side of the nose, and forms part of its lateral boundary.

*Nasal bones:* The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone:* The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit:* The bony cavity in the skull to contain the eyeball.

*Parietal bones:* The parietal bones are the bones that, when joined together, form the roof and sides of the cranium.

*Temporal bones:* The temporal bones are situated on the bases and sides of the skull, and support that part of the face known as the temple.

*Zygomatic bones:* The face includes two zygomatic bones, located in the upper and lateral parts of the face and forming the prominence of the cheek.

### 5.5.3.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.5.4 Patient interface

*Anti-asphyxia valve (AAV):* The component or sub-assembly of a mask system that, by opening to atmosphere in a failsafe manner, reduces the risk of excessive CO₂ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.5.5 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

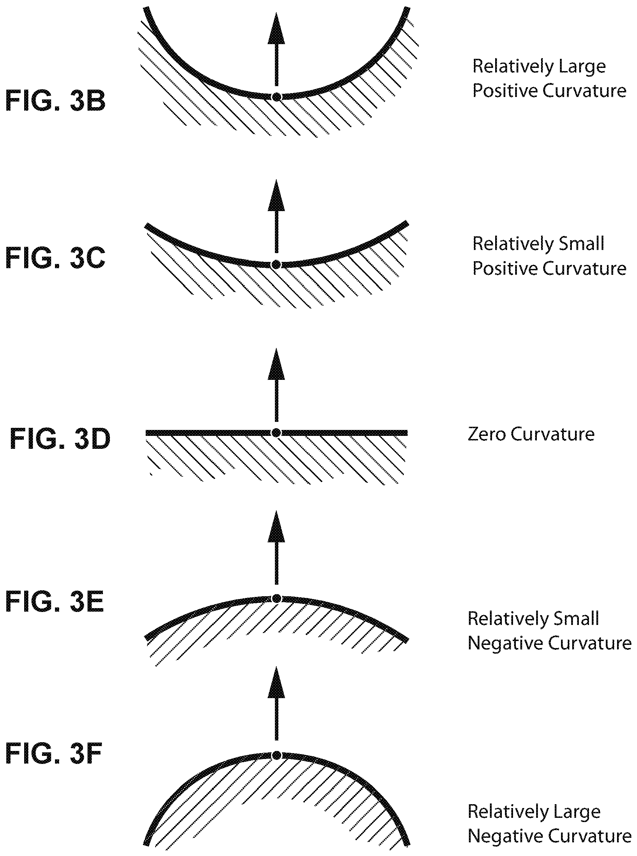

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 5.5.5.1 Curvature in one dimension

The curvature of a plane curve at *p* may be described as having a sign (e.g. positive, negative) and a magnitude (e.g. 1/radius of a circle that just touches the curve at *p*).

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 5.5.5.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from *f*(0) to *f*(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from *f*(0) to *f*(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 5.5.5.3 Space curves

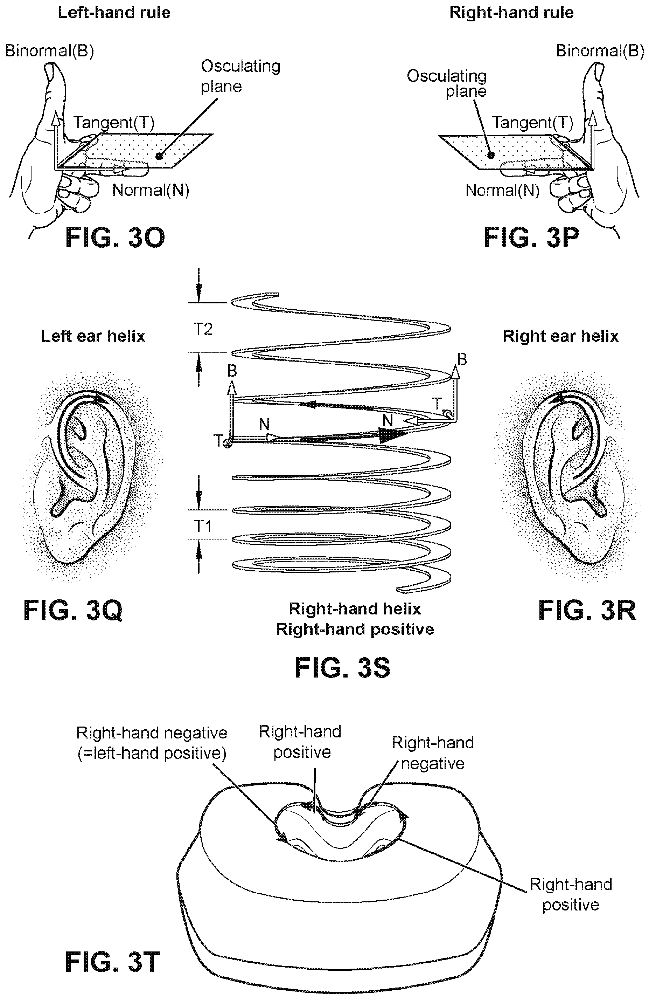

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S.

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 5.5.5.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 5.6 OTHER REMARKS

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

### 5.7 REFERENCE SIGNS LIST

| **Feature Item** | **Number** |
|---|---|
| patient | 1000 |
| bed partner | 1100 |
| patient interface | 3000 |
| seal - forming structure | 3100 |
| plenum chamber | 3200 |
| shell | 3205 |
| recessed portion | 3207 |
| chord | 3210 |
| superior point | 3220 |
| inferior point | 3230 |
| inlet port | 3240 |
| opening | 3250 |
| positioning and stabilising structure | 3300 |
| upper strap | 3310 |
| lower strap | 3320 |
| connection point | 3325 |
| clip | 3326 |
| headgear tube | 3340 |
| tab | 3342 |
| headgear tube connector | 3344 |
| conduit headgear inlet | 3390 |
| vent | 3400 |
| connection port | 3600 |
| forehead support | 3700 |
| RPT device | 4000 |
| air circuit | 4170 |
| AAV arrangement | 4400 |
| lateral opening | 4450 |
| shell portion | 4500 |
| port | 4510 |
| port openings | 4511 |
| vertical support | 4520 |
| horizontal support | 4530 |
| support member | 4540 |
| diffusing member | 4600 |
| diffusing member cover | 4700 |
| anterior wall | 4710 |
| side wall | 4720 |
| opening | 4750 |
| AAV member | 4800 |
| retaining portion | 4805 |
| flap portion | 4810 |
| hinge portion | 4815 |
| vent holes | 4830 |
| vent portion | 4840 |
| AAV guard | 4900 |
| wall | 4910 |
| humidifier | 5000 |
| AAV arrangement | 5400 |
| vent holes | 5405 |
| port | 5510 |
| supports | 5520 |
| AAV member | 5800 |
| retaining portion | 5805 |
| flap portion | 5810 |
| hinge portion | 5815 |
| surface | 5900 |
| AAV arrangement | 6400 |
| vent holes | 6405 |
| port | 6510 |
| port | 6512 |
| supports | 6520 |
| AAV member | 6800 |
| AAV guard | 6900 |
| wall | 6910 |

## Claims

1. A patient interface (3000) to deliver a flow of air at a positive pressure with respect to ambient air pressure to an entrance to the patient's airways including at least the entrance of a patient's nares while the patient (1000) is sleeping, to ameliorate sleep disordered breathing, the patient interface (3000) comprising:
a seal-forming structure (3100) constructed and arranged to form a seal with a region of a patient's face surrounding the entrance to the patient's airways, the seal-forming structure (3100) forming at least a portion of a plenum chamber pressurizable to a therapeutic pressure; and
a vent (3400) and an anti-asphyxia valve, AAV, arrangement (4400) configured to regulate flow therethrough to (1) provide a vent flow path when pressure in the plenum chamber (3200) is above a predetermined magnitude and (2) provide a breathable flow path when pressure in the plenum chamber (3200) is below the predetermined magnitude or not delivered,
the vent (3400) and AAV arrangement (4404) comprising:
a shell portion (4500) including a port extending therethrough, the port configured to allow gas to flow between the plenum chamber (3200) and ambient;
an AAV member (4800, 5800, 6800) provided to the shell portion (4500), **characterized in that** the AAV member (4800, 5800, 6800) includes a flap portion (4810, 5810) structured and arranged to regulate flow through the port,
wherein the flap portion (4810, 5810) includes a plurality of vent holes (4830, 5405, 6405) therethrough,
wherein the flap portion (4810, 5810) is movable to a deactivated position when pressure in the plenum chamber (3200) is above the predetermined magnitude to cover the port so that a vent flow of gas is allowed to pass along the vent flow path that extends through the plurality of vent holes (4830, 5405, 6405) of the flap portion (4810, 5810) and through the port, and
wherein the flap portion (4810, 5810) is movable to an activated position when pressure in the plenum chamber (3200) is below the predetermined magnitude or not delivered to uncover the port so that a breathable flow of gas is allowed to pass along the breathable flow path that extends through the port;
a diffusing member (4600) provided to the shell portion (4500), the diffusing member (4600) configured and arranged such that the port is covered by the diffusing member (4600) so that at least a portion of the vent flow of gas passes into the diffusing member (4600); and
a diffusing member cover (4700) to retain the diffusing member (4600) to the shell portion (4500), the diffusing member cover (4700) including an anterior wall (4710) completely covering an anterior side of the diffusing member (4600),
wherein the diffusing member (4600) and diffusing member cover (4700) are supported in spaced relation from an outlet end of the port to form a lateral opening (4450) between the shell portion (4500) and the diffusing member (4600) and diffusing member cover (4700), and
wherein at least a portion of the vent flow of gas passes into the diffusing member (4600) along the vent flow path and then is redirected laterally by the anterior wall (4710) to flow through the lateral opening (4450) to ambient.

2. The patient interface (3000) according to claim 1, further comprising a shell (3205) to support the seal-forming structure (3100) and form at least a portion of the plenum chamber (3200), wherein the shell portion (4500) of the vent (3400) and AAV arrangement is provided to the shell (3205).

3. The patient interface (3000) according to claim 2, wherein the shell portion (4500) forms an integral, one piece molded construction with the shell (3205).

4. The patient interface (3000) according to any one of claims 1 to 3, wherein the diffusing member (4600) comprises a textile material.

5. The patient interface (3000) according to any one of claims 1 to 4, further comprising an AAV guard (4900, 6900) provided to the shell portion (4500), the AAV guard (4900, 6900) forming a stop for the flap portion (4810, 5810) when in the activated position.

6. The patient interface (3000) according to claim 5, wherein the AAV guard (4900, 6900) comprises a wall oriented at an angle to the shell portion (4500).

7. The patient interface (3000) according to claim 6, wherein the wall is a solid wall without any holes therethrough.

8. The patient interface (3000) according to any one of claims 1 to 7, wherein at least the flap portion (4810, 5810) comprises a plastic material.

9. The patient interface (3000) according to any one of claims 1 to 8, wherein the AAV member (4800, 5800, 6800) comprises a silicone material.

10. The patient interface (3000) according to any one of claims 1 to 9, wherein the flap portion (4810, 5810) includes a textile vent or a microvent providing the plurality of vent holes (4830, 5405, 6405).

11. A CPAP system for providing gas at positive pressure for respiratory therapy to a patient (1000), the CPAP system comprising:
an RPT device (4000) configured to supply a flow of gas at a therapeutic pressure;
the patient interface (3000) according to any one of claims 1 to 10; and
an air delivery conduit configured to pass the flow of gas at the therapeutic pressure from the RPT device (4000) to the patient interface (3000).

## Patentansprüche

1. Patientenschnittstelle (3000) zur Abgabe einer Luftströmung mit einem Überdruck im Hinblick auf den Umgebungsluftdruck zu einem Eingang zu den Patientenluftwegen, einschließlich mindestens zum Eingang der Nasenlöcher eines Patienten, während der Patient (1000) schläft, um schlafbezogene Atmungsstörungen zu mildern, wobei die Patientenschnittstelle (3000) aufweist:
eine dichtungsbildende Struktur (3100), die so aufgebaut und angeordnet ist, dass sie eine Dichtung mit einer Region des Gesichts eines Patienten bildet, die den Eingang zu den Patientenluftwegen umgibt, wobei die dichtungsbildende Struktur (3100) mindestens einen Abschnitt einer Plenumkammer bildet, die auf einen Therapiedruck druckbeaufschlagbar ist; und
eine Entlüftungs- (3400) und Anti-Asphyxieventil- (AAV) Anordnung (4400), die so konfiguriert ist, dass sie eine Strömung durch sie hindurch reguliert, um (1) einen Entlüftungsströmungsweg bereitzustellen, wenn der Druck in der Plenumkammer (3200) über einer vorbestimmten Größe liegt, und (2) einen Atemströmungsweg bereitzustellen, wenn der Druck in der Plenumkammer (3200) unter der vorbestimmten Größe liegt oder nicht abgegeben wird,
wobei die Entlüftungs- (3400) und AAV-Anordnung (4404) aufweist:
einen Schalenabschnitt (4500) mit einem sich durch ihn erstreckenden Anschluss, wobei der Anschluss so konfiguriert ist, dass Gas zwischen der Plenumkammer (3200) und der Umgebung strömen kann;
ein AAV-Teil (4800, 5800, 6800), das am Schalenabschnitt (4500) vorgesehen ist,
**dadurch gekennzeichnet, dass** das AAV-Teil (4800, 5800, 6800) einen Klappenabschnitt (4810, 5810) aufweist, der so strukturiert und angeordnet ist, dass er die Strömung durch den Anschluss reguliert,
wobei der Klappenabschnitt (4810, 5810) mehrere durchgehende Entlüftungslöcher (4830, 5405, 6405) aufweist,
wobei der Klappenabschnitt (4810, 5810) in eine deaktivierte Position beweglich ist, wenn der Druck in der Plenumkammer (3200) über der vorbestimmten Größe liegt, um den Anschluss zu bedecken, so dass eine Entlüftungsgasströmung entlang des Entlüftungsströmungswegs passieren kann, der sich durch die mehreren Entlüftungslöcher (4830, 5405, 6405) des Klappenabschnitts (4810, 5810) und durch den Anschluss erstreckt, und
wobei der Klappenabschnitt (4810, 5810) in eine aktivierte Position beweglich ist, wenn der Druck in der Plenumkammer (3200) unter der vorbestimmten Größe liegt oder nicht abgegeben wird, um den Anschluss freizulegen, so dass eine Atemgasströmung entlang des Atemströmungswegs passieren kann, der sich durch den Anschluss erstreckt;
ein Diffusorelement (4600), das am Schalenabschnitt (4500) vorgesehen ist, wobei das Diffusorelement (4600) so konfiguriert und angeordnet ist, dass der Anschluss durch das Diffusorelement (4600) bedeckt ist, so dass mindestens ein Anteil der Entlüftungsgasströmung in das Diffusorelement (4600) strömt; und
eine Diffusorelementabdeckung (4700), um das Diffusorelement (4600) am Schalenabschnitt (4500) festzuhalten, wobei die Diffusorelementabdeckung (4700) eine anterior gelegene Wand (4710) aufweist, die eine anterior gelegene Seite des Diffusorelements(4600) vollständig bedeckt,
wobei das Diffusorelement (4600) und die Diffusorelementabdeckung (4700) in einer Abstandsbeziehung von einem Auslassende des Anschlusses gestützt werden, um eine lateral gelegene Öffnung (4450) zwischen dem Schalenabschnitt (4500) und dem Diffusorelement (4600) sowie der Diffusorelementabdeckung (4700) zu bilden, und
wobei mindestens ein Anteil der Entlüftungsgasströmung in das Diffusorelement (4600) entlang des Entlüftungsströmungswegs strömt und dann durch die anterior gelegene Wand (4710) lateral umgeleitet wird, um durch die lateral gelegene Öffnung (4450) in die Umgebung zu strömen.

2. Patientenschnittstelle (3000) nach Anspruch 1, ferner mit einer Schale (3205), um die dichtungsbildende Struktur (3100) zu stützen und mindestens einen Abschnitt der Plenumkammer (3200) zu bilden, wobei der Schalenabschnitt (4500) der Entlüftungs- (3400) und AAV-Anordnung an der Schale (3205) vorgesehen ist.

3. Patientenschnittstelle (3000) nach Anspruch 2, wobei der Schalenabschnitt (4500) einen integralen, einteiligen geformten Aufbau mit der Schale (3205) bildet.

4. Patientenschnittstelle (3000) nach einem der Ansprüche 1 bis 3, wobei das Diffusorelement (4600) ein Textilmaterial aufweist.

5. Patientenschnittstelle (3000) nach einem der Ansprüche 1 bis 4, ferner mit einem AAV-Schutz (4900, 6900), der am Schalenabschnitt (4500) vorgesehen ist, wobei der AAV-Schutz (4900, 6900) einen Anschlag für den Klappenabschnitt (4810, 5810) in der aktivierten Position bildet.

6. Patientenschnittstelle (3000) nach Anspruch 5, wobei der AAV-Schutz (4900, 6900) eine Wand aufweist, die in einem Winkel zum Schalenabschnitt (4500) orientiert ist.

7. Patientenschnittstelle (3000) nach Anspruch 6, wobei die Wand eine massive Wand ohne durchgehende Löcher ist.

8. Patientenschnittstelle (3000) nach einem der Ansprüche 1 bis 7, wobei mindestens der Klappenabschnitt (4810, 5810) ein Kunststoffmaterial aufweist.

9. Patientenschnittstelle (3000) nach einem der Ansprüche 1 bis 8, wobei das AAV-Teil (4800, 5800, 6800) ein Silikonmaterial aufweist.

10. Patientenschnittstelle (3000) nach einem der Ansprüche 1 bis 9, wobei der Klappenabschnitt (4810, 5810) eine Textilentlüftung oder eine Mikroentlüftung aufweist, die die mehreren Entlüftungslöcher (4830, 5405, 6405) bereitstellt.

11. CPAP-System (Continuous Positive Airway Pressure System) zur Bereitstellung von Gas mit Überdruck zur Atemtherapie eines Patienten (1000), wobei das CPAP-System aufweist:
eine Atemdrucktherapie- (RPT-) Vorrichtung (4000), die so konfiguriert ist, dass sie eine Gasströmung mit einem Therapiedruck zuführt;
die Patientenschnittstelle (3000) nach einem der Ansprüche 1 bis 10; und
eine Luftabgabeleitung, die so konfiguriert ist, dass sie die Gasströmung mit dem Therapiedruck von der RPT-Vorrichtung (4000) zur Patientenschnittstelle (3000) führt.

## Revendications

1. Interface patient (3000) pour délivrer un flux d'air à une pression positive par rapport à la pression de l'air ambiant à une entrée des voies respiratoires d'un patient incluant au moins l'entrée des narines d'un patient pendant que le patient (1000) dort, pour améliorer la respiration anormale du sommeil, l'interface patient (3000) comprenant :
une structure formant joint d'étanchéité (3100) construite et agencée pour former un joint d'étanchéité avec une région du visage d'un patient entourant l'entrée des voies respiratoires du patient, la structure formant joint d'étanchéité (3100) formant au moins une partie d'une chambre de distribution pouvant être pressurisée à une pression thérapeutique ; et
un agencement d'évent (3400) et de valve anti-asphyxie, VAA, (4400) configuré pour réguler le flux à travers celui-ci pour (1) fournir un trajet d'écoulement d'évent lorsque la pression dans la chambre de distribution (3200) est au-dessus d'une grandeur prédéterminée et (2) fournir un trajet d'écoulement respirable lorsque la pression dans la chambre de distribution (3200) est en dessous de la grandeur prédéterminée ou n'est pas délivrée,
l'agencement d'évent (3400) et de VAA (4404) comprenant :
une partie coque (4500) incluant un orifice s'étendant à travers celle-ci, l'orifice étant configuré pour permettre au gaz de s'écouler entre la chambre de distribution (3200) et l'air ambiant,
un élément de VAA (4800, 5800, 6800) prévu sur la partie coque (4500), **caractérisée en ce que** l'élément de VAA (4800, 5800, 6800) inclut
une partie volet (4810, 5810) structurée et agencée pour réguler le flux à travers l'orifice,
dans laquelle la partie volet (4810, 5810) inclut une pluralité de trous d'évent (4830, 5405, 6405) à travers celle-ci,
dans laquelle la partie volet (4810, 5810) est mobile vers une position désactivée lorsque la pression dans la chambre de distribution (3200) est au-dessus de la grandeur prédéterminée pour couvrir l'orifice de sorte qu'un flux d'évent de gaz est autorisé à passer suivant le trajet d'écoulement d'évent qui s'étend à travers la pluralité de trous d'évent (4830, 5405, 6405) de la partie volet (4810, 5810) et à travers l'orifice, et
dans laquelle la partie volet (4810, 5810) est mobile vers une position activée lorsque la pression dans la chambre de distribution (3200) est en dessous de la grandeur prédéterminée ou n'est pas délivrée pour découvrir l'orifice de sorte qu'un flux respirable de gaz est autorisé à passer suivant le trajet d'écoulement respirable qui s'étend à travers l'orifice,
un élément de diffusion (4600) prévu sur la partie coque (4500), l'élément de diffusion (4600) étant configuré et agencé de telle sorte que l'orifice est couvert par l'élément de diffusion (4600) de sorte qu'au moins une partie du flux d'évent de gaz passe dans l'élément de diffusion (4600) ; et
un couvercle d'élément de diffusion (4700) pour retenir l'élément de diffusion (4600) sur la partie coque (4500), le couvercle d'élément de diffusion (4700) incluant une paroi antérieure (4710) couvrant complètement un côté antérieur de l'élément de diffusion (4600),
dans laquelle l'élément de diffusion (4600) et le couvercle d'élément de diffusion (4700) sont supportés en relation espacée d'une extrémité de sortie de l'orifice pour former une ouverture latérale (4450) entre la partie coque (4500) et l'élément de diffusion (4600) et le couvercle d'élément de diffusion (4700), et
dans laquelle au moins une partie du flux d'évent de gaz passe dans l'élément de diffusion (4600) suivant le trajet d'écoulement d'évent et est ensuite redirigée latéralement par la paroi antérieure (4710) pour s'écouler à travers l'ouverture latérale (4450) vers l'air ambiant.

2. Interface patient (3000) selon la revendication 1, comprenant en outre une coque (3205) pour supporter la structure formant joint d'étanchéité (3100) et former au moins une partie de la chambre de distribution (3200), dans laquelle la partie coque (4500) de l'agencement d'évent (3400) et de VAA est prévue sur la coque (3205).

3. Interface patient (3000) selon la revendication 2, dans laquelle la partie coque (4500) forme une construction moulée d'un seul tenant, intégrale avec la coque (3205).

4. Interface patient (3000) selon l'une quelconque des revendications 1 à 3, dans laquelle l'élément de diffusion (4600) comprend une matière textile.

5. Interface patient (3000) selon l'une quelconque des revendications 1 à 4, comprenant en outre une protection de VAA (4900, 6900) prévue sur la partie coque (4500), la protection de VAA (4900, 6900) formant une butée pour la partie volet (4810, 5810) lorsqu'elle est dans la position activée.

6. Interface patient (3000) selon la revendication 5, dans laquelle la protection de VAA (4900, 6900) comprend une paroi orientée selon un angle par rapport à la partie coque (4500).

7. Interface patient (3000) selon la revendication 6, dans laquelle la paroi est une paroi pleine sans aucun trou à travers celle-ci.

8. Interface patient (3000) selon l'une quelconque des revendications 1 à 7, dans laquelle au moins la partie volet (4810, 5810) comprend une matière plastique.

9. Interface patient (3000) selon l'une quelconque des revendications 1 à 8, dans laquelle l'élément de VAA (4800, 5800, 6800) comprend une matière de silicone.

10. Interface patient (3000) selon l'une quelconque des revendications 1 à 9, dans laquelle la partie volet (4810, 5810) inclut un évent textile ou un micro-évent fournissant la pluralité de trous d'évent (4830, 5405, 6405).

11. Système CPAP pour fournir un gaz à pression positive pour une thérapie respiratoire à un patient (1000), le système CPAP comprenant :
un dispositif RPT (4000) configuré pour fournir un flux de gaz à une pression thérapeutique ;
l'interface patient (3000) selon l'une quelconque des revendications 1 à 10 ; et
un conduit d'amenée d'air configuré pour faire passer le flux de gaz à la pression thérapeutique du dispositif RPT (4000) à l'interface patient (3000).
